# EUROPEAN PATENT APPLICATION

(11) **EP 4 563 585 A1**
(43) Date of publication of application: **04.06.2025**
(21) Application number: 23845523.2
(22) Date of filing: 25.07.2023
(51) Int. Cl.: C07K 1/107, C07K 1/10, C07D 401/14

(54) **METHOD FOR SITE-DIRECTED SYNTHESIS OF PROTEIN-ACTIVE MOLECULAR CONJUGATE**

(30) Priority: 25.07.2022 CN 202210880480
(71) Applicant: Nanjing GenScript Biotech Co., Ltd., Nanjing, Jiangsu 211100 (CN)
(72) Inventor: GUO, Chun, Nanjing, Jiangsu 211100 (CN); XU, Kang, Nanjing, Jiangsu 211100 (CN); CHEN, Chen, Nanjing, Jiangsu 211100 (CN); WANG, Jianpeng, Nanjing, Jiangsu 211100 (CN); LI, Hong, Nanjing, Jiangsu 211100 (CN)
(74) Representative: Wichmann, Hendrik
(86) International application number: PCT/CN2023/109032
(87) International publication number: WO 2024/022313

(57) **Abstract**

Provided is a bioconjugation technique, specifically provided is a protein covalent modification technique, i.e., provided is a protein moiety-linker conjugate, which can be used as an intermediate for synthesizing a protein-active molecule conjugate. Further provided are a method for preparing the protein moiety-linker conjugate, and a method for synthesizing a protein-active molecule conjugate by means of using the protein moiety-linker conjugate as an intermediate. The method has a higher conversion rate and fewer by-products.

## Description

### CROSS REFERENCE TO RELATED APPLICATION

The present application claims the right of priority for Chinese Patent Application No. CN 202210880480.X filed on July 25, 2022, which is incorporated herein by reference in its entirety.

### TECHNICAL FIELD

The present invention relates to a bioconjugation technique. In particular, the present invention relates to a protein covalent modification technique, that is, the present invention provides a protein moiety-linker conjugate, which can be used as an intermediate for synthesizing a protein-active molecule conjugate. The present invention further relates to a method for preparing the protein moiety-linker conjugate, and a method for synthesizing a protein-active molecule conjugate by means of using the protein moiety-linker conjugate as an intermediate. The method of the present invention has a higher conversion rate and fewer by-products.

### BACKGROUND ART

Chemical modification of proteins has a wide range of biological applications. In recent years, small molecule modifications have been applied to protein analysis (Nature Protocols 2007, 2 (6), 1414-1425) and cell imaging (Nature Methods 2006, 3 (8), 591-596). Biopolymer modifications contribute to the rational design of protein drugs (Chemical Society Reviews 2018, 47 (24), 8998-9014). In addition, inspired by the successful application of antibody-drug conjugates (ADCs), antibody-oligo conjugates (AOCs) also have application prospects in the field of targeted delivery oligonucleotides (J Control Release 2016, 237, 1-13).

Covalent modification of proteins while maintaining their functions has been a goal of chemical biology research. Generally, lysine and cysteine are selected for the modification of proteins due to their reactive side chains. However, the overall frequency of lysine in proteins is about 5.9%, and without accurate control of labeling amount and sites, this method usually results in a mixture of multiple proteins with site modifications and even changes the original biological function of the protein (Nature Biotechnology 2008, 26 (8), 925-932). Cysteine, on the other hand, generally maintains the active protein structure by forming disulfide bonds, and modifications to cysteine sites may be detrimental to maintaining proper folding of the protein (Trends in biochemical sciences 2003, 28 (4), 210-4). To avoid these problems, site-specific modification methods such as amber codon suppression (ACS chemical biology 2019, 14 (4), 603-612), protein end labeling (Nature Protocols 2013, 8 (9), 1800-1807), enzyme labeling (The Journal of biological chemistry 2017, 292 (38), 15622-15635), and affinity tag-mediated labeling (Angewandte Chemie International Edition 2019, 58 (17), 5592-5597) have been reported. However, these methods often require intervention in the complex translation process of proteins to insert reaction sites or mediation of enzymes.

The conversion rates of existing macromolecular modifications of proteins by 2-picolinaldehyde derivatives are mostly only about 50%, and there are by-products generated after multiple addition reactions (Bioconjugate Chemistry 2019, 30 (9), 2427-2434). Moreover, the modification products are less stable, among which the product with only a 2-picolinaldehyde modification degrades by about 30% after 12 h of incubation at 37°C (Nature Chemical Biology 2015, 11 (5), 326-331).

Therefore, there is an urgent need in the art for a novel method for synthesizing a protein-nucleic acid conjugate, which has advantages such as a higher conversion rate and fewer by-products compared with existing methods.

### SUMMARY OF THE INVENTION

An objective of the present invention is to provide a protein moiety-linker conjugate that can be used as an intermediate for synthesizing a protein-active molecule conjugateprotein-active molecule conjugate, a method for preparing the protein moiety-linker conjugate, and a method for synthesizing a protein-nucleic acid conjugate by means of using the protein moiety-linker conjugate as an intermediate. According to the method of the present invention, on the basis of the property that a 2-picolinaldehyde derivative specifically labels the nitrogen terminus (N-terminus) of a protein, a protein moiety-linker conjugate is generated as an intermediate under the mediation of a polyhistidine tag (His tag) located at the N-terminus and a maleimide derivative, thereby further obtaining a protein-active molecule conjugate. The method of the present invention has a higher conversion rate and fewer by-products compared to the previously reported method (mAbs 2018, 10 (5), 712-719). In the present invention, there is a significant improvement in both conversion rate and the stability of conjugate on the basis of maintaining site-directed conjugation, with the conversion rate being increased to 80% or more, and the degradation rate of the conjugate being less than 5%.

In one aspect, the present invention provides a protein moiety-linker conjugate having a structure as shown in formula (I): 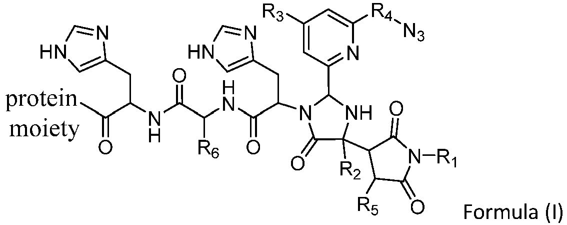
wherein R₁ is selected from hydrogen, hydrocarbyl, amine group, amino, hydroxyl, carboxyl, the hydrocarbyl optionally has one or more substituents selected from hydrocarbyl, amine group, amino, hydroxyl, carboxyl, and the hydrocarbyl is preferably alkyl, aryl, or a combination thereof, wherein the alkyl optionally has one or more identical or different substituents selected from aryl, amine group, amino, hydroxyl, carboxyl, and the aryl optionally has one or more identical or different substituents selected from alkyl, amine group, amino, hydroxyl, carboxyl, or R₁ is selected from a fluorophore, a PEG modification group, a biotin modification group, a polypeptide modification group, a nucleic acid modification group;
R₂ is selected from a side chain moiety of any one of natural amino acids;
R₃ is selected from hydrogen, alkyl, alkoxy, hydroxyl, amino, amine group;
R₄ is selected from alkylene, arylene, PEG;
R₅ is selected from hydrogen and alkyl;
R₆ is selected from a side chain moiety of any one of natural amino acids.

In some embodiments, the protein moiety-linker conjugate of the present invention has the structure of:

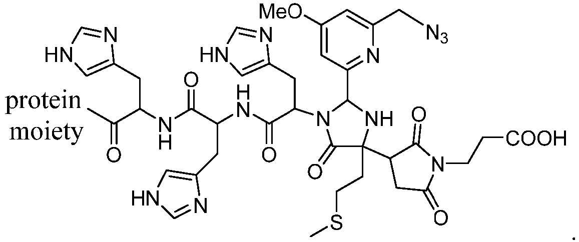

In some embodiments, in the protein moiety-linker conjugate of the present invention, the protein moiety is an antibody or a fragment thereof.

In some embodiments, in the protein moiety-linker conjugate of the present invention, the protein moiety has a molecular weight ranging from 8 kDa to 160 kDa.

In one aspect, the present invention provides a method for preparing a protein moiety-linker conjugate, which method comprises the steps of:
(1) modifying the N-terminus of a protein using a short peptide containing His tag to obtain a modified protein;
(2) subjecting the modified protein to a reaction with an azido-containing 2-picolinaldehyde derivative and a maleimide derivative to obtain the protein moiety-linker conjugate.

In some embodiments, in the method for preparing a protein moiety-linker conjugate of the present invention, the short peptide containing His tag has a sequence of Xaa-His-Xaa-His, wherein Xaa is any one of natural amino acid residues.

In some embodiments, in the method for preparing a protein moiety-linker conjugate of the present invention, the azido-containing 2-picolinaldehyde derivative is a 6-(azidomethyl)-2-picolinaldehyde derivative, preferably 6-(azidomethyl)-4-methoxypicolinaldehyde.

In some embodiments, in the method for preparing a protein moiety-linker conjugate of the present invention, the maleimide derivative has the structure of: 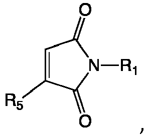
wherein R₁ is selected from hydrogen, hydrocarbyl, amine group, amino, hydroxyl, carboxyl, the hydrocarbyl optionally has one or more substituents selected from hydrocarbyl, amine group, amino, hydroxyl, carboxyl, and the hydrocarbyl is preferably alkyl, aryl, or a combination thereof, wherein the alkyl optionally has one or more identical or different substituents selected from aryl, amine group, amino, hydroxyl, carboxyl, and the aryl optionally has one or more identical or different substituents selected from alkyl, amine group, amino, hydroxyl, carboxyl, or R₁ is a fluorophore, a PEG modification group, a biotin modification group, a polypeptide modification group, a nucleic acid modification group;
R₅ is selected from hydrogen and alkyl.

In some embodiments, in the method for preparing a protein moiety-linker conjugate of the present invention, the maleimide derivative is selected from

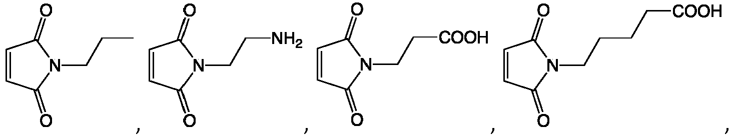

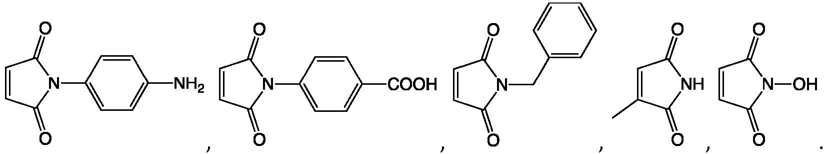

In some embodiments, in the method for preparing a protein moiety-linker conjugate of the present invention, the modified protein, the azido-containing 2-picolinaldehyde derivative, and the maleimide derivative react in a molar ratio of 1 : 5-60 : 5-40.

In some embodiments, in the method for preparing a protein moiety-linker conjugate of the present invention, the molar ratio of the azido-containing 2-picolinaldehyde derivative to the maleimide derivative is in the range of 30 : 1 to 1 : 1.

In some embodiments, in the method for preparing a protein moiety-linker conjugate of the present invention, the reaction of the modified protein with the azido-containing 2-picolinaldehyde derivative and the maleimide derivative is performed at pH 6.0-9.0.

In one aspect, the present invention provides the use of the protein moiety-linker conjugate in the preparation of a protein-active molecule conjugate.

In some embodiments, in the use of the protein moiety-linker conjugate in the preparation of a protein-active molecule conjugate of the present invention, the active molecule is selected from a nucleic acid molecule, a fluorescent molecule, a PEG molecule, a biotin molecule, a polypeptide molecule, a small drug molecule.

In one aspect, the present invention provides a method for synthesizing a protein-active molecule conjugate, which method comprises the steps of:
subjecting the protein moiety-linker conjugate of the present invention to a reaction with an alkynyl-modified active molecule to obtain the protein-active molecule conjugate.

In some embodiments, in the method for synthesizing a protein-active molecule conjugate of the present invention, the active molecule is selected from a nucleic acid molecule, a fluorescent molecule, a PEG molecule, a biotin molecule, a polypeptide molecule, a small drug molecule.

In some embodiments, in the method for synthesizing a protein-active molecule conjugate of the present invention, the active molecule is a nucleic acid molecule.

In some embodiments, in the method for synthesizing a protein-active molecule conjugate of the present invention, the alkynyl-modified active molecule is a nucleic acid molecule with an alkynyl-containing group modification at the 3' end, the 5' end, or any position between the two ends.

In some embodiments, in the method for synthesizing a protein-active molecule conjugate of the present invention, the alkynyl-modified active molecule is a nucleic acid molecule with a dibenzocyclooctyne modification at the 3' end or the 5' end or any position between the two ends.

In some embodiments, in the method for synthesizing a protein-active molecule conjugate of the present invention, the nucleic acid molecule is selected from natural DNA; natural RNA; a nucleic acid having a base modification such as 2'-F modification, 2'-OMe modification, and 2'-MOE modification; a nucleic acid having a biotin modification, a fluorophore modification, a polypeptide modification, a PEG modification, a phosphorylation modification; the nucleic acid molecule includes a single-stranded nucleic acid or a double-stranded nucleic acid.

In some embodiments, in the method for synthesizing a protein-active molecule conjugate of the present invention, the nucleic acid molecule has a length of 10 to 100 nt.

In one aspect, the present invention provides a protein-active molecule conjugate obtained by the method for synthesizing a protein-active molecule conjugate of the present invention.

In some embodiments, in the protein-active molecule conjugate obtained by the method for synthesizing a protein-active molecule conjugate of the present invention, the active molecule is selected from a nucleic acid molecule, a fluorescent molecule, a PEG molecule, a biotin molecule, a polypeptide molecule, a small drug molecule.

In some embodiment, in the protein-active molecule conjugate obtained by the method of synthesizing a protein-active molecule conjugate of the present invention, the active molecule is a nucleic acid molecule.

In one aspect, the present invention provides a protein-nucleic acid conjugate, which has the structure of: 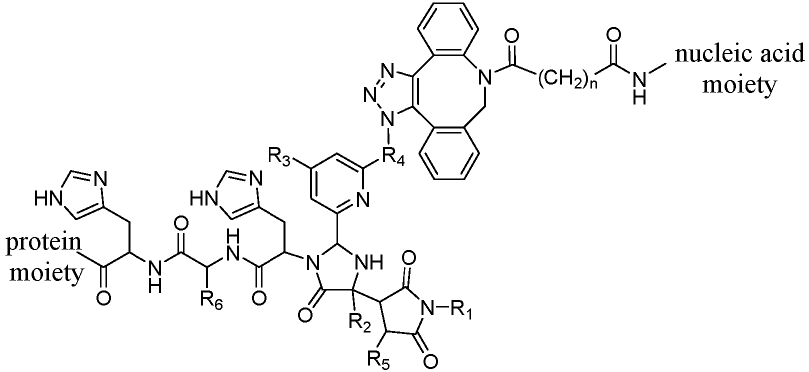
wherein R₁ is selected from hydrogen, hydrocarbyl, amine group, amino, hydroxyl, carboxyl, the hydrocarbyl optionally has one or more substituents selected from hydrocarbyl, amine group, amino, hydroxyl, carboxyl, and the hydrocarbyl is preferably alkyl, aryl, or a combination thereof, wherein the alkyl optionally has one or more identical or different substituents selected from aryl, amine group, amino, hydroxyl, carboxyl, and the aryl optionally has one or more identical or different substituents selected from alkyl, amine group, amino, hydroxyl, carboxyl, or R₁ is selected from a fluorophore, a PEG modification group, a biotin modification group, a polypeptide modification group, a nucleic acid modification group;
R₂ is selected from a side chain moiety of any one of natural amino acids;
R₃ is selected from hydrogen, alkyl, alkoxy, hydroxyl, amino, amine group;
R₄ is selected from alkylene, arylene, PEG;
R₅ is selected from hydrogen and alkyl;
R₆ is selected from a side chain moiety of any one of natural amino acids;
n is an integer selected from 1-8.

In some embodiments, the protein-nucleic acid conjugate of the present invention has the structure of:

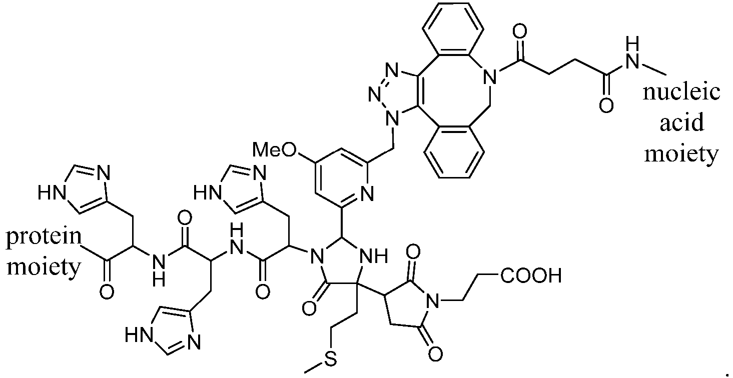

In some embodiments, in the protein-nucleic acid conjugate of the present invention, the protein moiety is an antibody or a fragment thereof.

In some embodiments, in the protein-nucleic acid conjugate of the present invention, the protein moiety has a molecular weight ranging from 8 kDa to 160 kDa.

In some embodiments, in the protein-nucleic acid conjugate of the present invention, the nucleic acid molecule is selected from natural DNA; natural RNA; a nucleic acid having a base modification such as 2'-F modification, 2'-OMe modification, and 2'-MOE modification; a nucleic acid having a biotin modification, a fluorophore modification, a polypeptide modification, a PEG modification, a phosphorylation modification; the nucleic acid molecule includes a single-stranded nucleic acid or a double-stranded nucleic acid.

In some embodiments, in the protein-nucleic acid conjugate of the present invention, the nucleic acid molecule has a length of 10 to 100 nt.

### BRIEF DESCRIPTION OF THE DRAWINGS

FIG. 1 shows a diagram of a synthetic route for the preparation of protein-nucleic acid conjugate according to the method of the present invention.
FIG. 2 shows a diagram of a preparative route for azido-containing 2-picolinaldehyde derivative as N-terminal labeling probe (NLP).
FIG. 3 shows a diagram of a synthetic route for oligonucleotide with 3'-end DBCO modification.
FIG. 4 shows an HPLC analysis profile of purified oligonucleotide with 3'-end DBCO modification.
FIG. 5 shows a mass spectrometry profile of oligonucleotide with 3'-end amino modification and 5'-end biotin modification.
FIG. 6 shows a mass spectrometry profile of oligonucleotide with 3'-end DBCO modification and 5'-end biotin modification.
FIG. 7 shows a diagram of a synthetic route for the preparation of protein moiety-linker conjugate according to the method of the present invention.
FIG. 8 shows mass spectrometry profiles of maltose-binding protein and maltose-binding protein with various modifications before, during, and after reaction, where a is mass spectrometry profile of maltose-binding protein; b is a mass spectrometry profile of maltose-binding protein with small azide molecule modification; c is a mass spectrometry profile of maltose-binding protein-nucleic acid conjugate.
FIG. 9 shows a liquid chromatography-mass spectrometry analysis profile of sample after enzymatic cleavage of protein-nucleic acid conjugate.
FIG. 10 shows the conjugation efficiency of maltose-binding protein modified with different maleimide derivative small molecules to nucleic acid.
FIG. 11 shows protein-nucleic acid conjugation efficiency after small molecule modifications in different pH solutions.
FIG. 12 shows protein-nucleic acid conjugation efficiency after modifications with different equivalents of small molecules.
FIG. 13 shows protein-nucleic acid conjugation efficiency after small molecule modifications in different buffer salt systems.
FIG. 14 shows protein-nucleic acid conjugation efficiency after the addition of different additives for small molecule modifications.
FIG. 15 shows the conjugation efficiency of maltose-binding protein modified with different maleimide derivative small molecules to nucleic acid and the proportion of conjugates after heating.
FIG. 16 shows the conjugation efficiency of green fluorescent protein, ubiquitin protein, small ubiquitin-like modifier protein modified with maleimide derivative small molecule to nucleic acid.
FIG. 17 shows an HPLC analysis profile of purified 65 nt long-chain oligonucleotide with 3'-end DBCO modification.
FIG. 18 shows a mass spectrometry profile of long-chain oligonucleotide with 3'-end amino modification and 5'-end biotin modification.
FIG. 19 shows a mass spectrometry profile of long-chain oligonucleotide with 3'-end DBCO modification and 5'-end biotin modification.
FIG. 20 shows the conjugation efficiency of maltose-binding protein to nucleic acids of different lengths.
FIG. 21 shows the modification proportion of the heavy chain of antibody-nucleic acid conjugate.
FIG. 22 shows Elisa assay of antibodies and obtained antibody-nucleic acid conjugates and the comparison of IC50 values.

### DETAILED DESCRIPTION OF EMBODIMENTS

Unless otherwise defined, all scientific and technical terms used herein have the same meaning as commonly understood by those of ordinary skill in the art to which the present invention belongs. Those skilled in the art of the present invention would understand that the methods and materials described below are exemplary only and should not be considered as limiting the scope of the present invention.

The inventors have found that a 6-(azidomethyl)-2-picolinaldehyde derivative can be linked to the N-terminus of a protein to form a protein moiety-linker conjugate under the co-mediation of a His tag modified and linked to the N-terminus of the protein and a maleimide derivative. In one aspect, the protein moiety-linker conjugate can be used as an intermediate to further be subjected to a linking reaction with an active molecule, e.g., a biomolecule, including a nucleic acid, or a small drug molecule, to form a protein-active molecule conjugate. This method improves the conversion rate and reduces the generation of by-products compared with the prior art. In a further aspect, the protein moiety-linker conjugate itself can be made to include an active molecule moiety by a maleimide derivative comprising the active molecule moiety, as will be described in more detail below.

Proteins that can be used in the method of the present invention are not particularly limited. As used herein, the term "protein" encompasses any type of protein, refers to any polymer of amino acids of any length, and thus also encompasses peptides, e.g., a peptide containing only 2 to 100 amino acids, whereby the term "protein" is used interchangeably with the term "polypeptide". As used herein, the term "protein" is generally used to describe a macromolecule comprising one amino acid chain, and also describes a macromolecule comprising multiple amino acid chains. In some embodiments, the present invention includes full-length proteins, and fragments or domains of proteins. In addition, as used herein, the term "protein" refers to a linear amino acid chain, or refers to an amino acid chain that has been processed and folded into a functional protein. In some embodiments, the protein can be obtained by isolation and purification from cells that naturally produce the protein, cleavage by an enzyme (e.g., a proteolytic enzyme), and/or by recombinant expression of a nucleic acid encoding the protein or fragment. In some embodiments, the protein and/or the fragment thereof of the present invention can be obtained by chemical synthesis or other known protocols for the production of proteins and fragments thereof. In some embodiments, the protein is preferably an antibody or a fragment thereof. In some embodiments, it is preferred that the protein has a molecular weight ranging from 8 kDa to 160 kDa and an isoelectric point of 5.0 to 9.5.

As used herein, the term "antibody" has a broad meaning and specifically includes monoclonal antibodies (including full-length antibodies having immunoglobulin Fc regions), antibody compositions having multi-epitope specificity, multispecific antibodies (e.g., bispecific antibodies, diabodies, and single chain molecules), and antibody fragments. The term "immunoglobulin" (Ig) is used interchangeably herein with "antibody". Herein, the antibody may be an antibody of any class (such as IgG, IgE, IgM, IgD, IgA) or subclass (such as IgG₁, IgG₂, IgG₂ₐ, IgG₃, IgG₄, IgA₁, IgA₂).

As used herein, the term "monoclonal antibody" refers to an antibody obtained from a population of substantially homogeneous antibodies, that is, the individual antibodies constituting the population are identical, except for possible naturally occurring mutations and/or post-translational modifications (e.g., isomerization, amidation) that may be present in minor amounts. Monoclonal antibodies are highly specific and therefore are directed against a single antigenic site.

As used herein, the term "antibody fragment" includes a portion of an intact antibody, preferably an antigen binding region and/or a variable region of the intact antibody. Examples of antibody fragments include Fab, Fab', F(ab')₂, and Fv fragments; diabodies; linear antibodies; nanobodies; single-chain antibody molecules and multispecific antibodies formed from antibody fragments.

In the present invention, the N-terminus of a protein is modified with a short peptide containing His tag.

In some embodiments, the short peptide containing His tag has a sequence of Xaa-His-Xaa-His, wherein Xaa is any one of natural amino acid residues. Xaa is not particularly limited and may be any one of natural amino acid residues. Thus, in the protein moiety-linker conjugate of the present invention, R₂ and R₆ may be side chain group moieties of any one of natural amino acids.

As used herein, the term "natural amino acid" refers to any one of common, naturally occurring amino acids found in naturally occurring proteins. Examples of natural amino acids include glycine, alanine, valine, leucine, isoleucine, methionine, proline, phenylalanine, tryptophan, serine, threonine, cysteine, tyrosine, asparagine, glutamine, aspartic acid, glutamic acid, lysine, arginine, histidine.

In some embodiments, the His tag moiety of the short peptide containing His tag is linked to the N-terminus of the protein.

In some embodiments, the residue Xaa in the short peptide containing His tag has a free -NH₂ group when linked to the N-terminus of the protein.

The linking of the short peptide containing His tag to the N-terminus of the protein can be performed by using methods known in the art, which are not particularly limited in the present application.

After obtaining a protein modified with a short peptide containing His tag, it reacts with an azido-containing 2-picolinaldehyde derivative and a maleimide derivative to obtain the protein moiety-linker conjugate of the present invention.

As used herein, the term "hydrocarbyl" refers to a group containing only hydrogen and carbon atoms, and the hydrocarbyl may contain one or more double and/or triple carbon-carbon bonds, and may contain a cyclic group attached to a linear or branched chain group. The hydrocarbyl may contain 1 to 20 carbon atoms. In some embodiments, the hydrocarbyl contains 1 to 20, 1 to 18, 1 to 16, 1 to 14, 1 to 12, 1 to 10, 1 to 8, 1 to 6, or 1 to 4 carbon atoms. Herein, the hydrocarbyl includes alkyl, alkenyl, cycloalkyl, cycloalkenyl, alkynyl, aryl, and combinations thereof.

As used herein, the term "alkyl" refers to a saturated hydrocarbyl group obtained from a saturated alkane losing one hydrogen atom. The alkyl may be linear, branched, cyclic, or a combination thereof. When the alkyl is cyclic, it may also be referred to as "cycloalkyl". The alkyl generally has 1 to 20 carbon atoms. In some embodiments, the alkyl contains 1 to 20, 1 to 18, 1 to 16, 1 to 14, 1 to 12, 1 to 10, 1 to 8, 1 to 6, or 1 to 4 carbon atoms. Non-limiting examples of alkyl include, but are not limited to, methyl, ethyl, propyl (including n-propyl, isopropyl), butyl (including n-butyl, isobutyl, sec-butyl, tert-butyl), n-pentyl, isopentyl, n-hexyl, cyclohexyl, n-heptyl, n-octyl, ethylhexyl, etc. In some embodiments, the alkyl includes methyl, ethyl, propyl, butyl, etc.

As used herein, the term "alkenyl" refers to an unsaturated hydrocarbyl group obtained from an unsaturated olefin losing one hydrogen atom, and has one or more carbon-carbon double bonds. The alkenyl generally has 1 to 20 carbon atoms. In some embodiments, the alkenyl contains 1 to 20, 1 to 18, 1 to 16, 1 to 14, 1 to 12, 1 to 10, 1 to 8, 1 to 6, or 1 to 4 carbon atoms. Non-limiting examples of alkenyl include, but are not limited to, ethenyl, propenyl, butenyl, pentenyl, and hexenyl.

As used herein, the term "alkynyl" refers to an unsaturated hydrocarbyl group obtained from an unsaturated alkyne losing one hydrogen atom, and has one or more carbon-carbon triple bonds. The alkynyl generally has 1 to 20 carbon atoms. In some embodiments, the alkynyl contains 1 to 20, 1 to 18, 1 to 16, 1 to 14, 1 to 12, 1 to 10, 1 to 8, 1 to 6, or 1 to 4 carbon atoms. Non-limiting examples of alkynyl include, but are not limited to, ethynyl, propynyl, butynyl, pentynyl, and hexynyl.

As used herein, the term "alkylene" refers to a saturated hydrocarbyl group obtained from a saturated alkane losing two hydrogen atoms. The alkylene may be linear, branched, cyclic, or a combination thereof. When the alkylene is cyclic, it may also be referred to as "cycloalkylene". The alkylene generally has 1 to 20 carbon atoms. In some embodiments, the alkylene contains 1 to 20, 1 to 18, 1 to 16, 1 to 14, 1 to 12, 1 to 10, 1 to 8, 1 to 6, or 1 to 4 carbon atoms. The alkylene may be obtained from a corresponding saturated alkane losing two hydrogen atoms on the same carbon atom or losing one hydrogen atom each on different carbon atoms. Non-limiting examples of alkyl include, but are not limited to, methylene (i.e., - CH₂-), ethylene (i.e., -CH₂CH₂- or -CH(CH₃)-), propylene (i.e., -CH₂CH₂CH₂-, -CH(CH₂CH₃)-, or - CH(CH₃)CH₂-), and butylene (i.e., -CH₂CH₂CH₂CH₂-, -CH(CH₃)CH₂CH₂-, -CH₂CH(CH₃)CH₂-, or - CH(CH₂CH₃)CH₂-).

As used herein, the term "alkoxy" refers to a group having the formula -OR, wherein R is alkyl directly linked to oxy. The alkyl is as defined above. The alkoxy generally has 1 to 20 carbon atoms. In some embodiments, the alkoxy contains 1 to 20, 1 to 18, 1 to 16, 1 to 14, 1 to 12, 1 to 10, 1 to 8, 1 to 6, or 1 to 4 carbon atoms. Non-limiting examples of alkoxy include methoxy, ethoxy, propoxy (including n-propoxy and isopropoxy), butoxy (including n-butoxy, isobutoxy, sec-butoxy, and tert-butoxy), pentoxy, hexoxy, etc. In some embodiments, the alkoxy includes methoxy, ethoxy, propoxy, butoxy, etc.

As used herein, the term "aryl" refers to an aromatic group containing one ring or two or three fused rings. The aryl may have one to five rings attached or fused to an aromatic ring. The other ring structures may be aromatic, non-aromatic, or combinations thereof. The aryl may have 6 to 20 carbon atoms. In some embodiments, the aryl contains 6 to 18, 6 to 16, 6 to 14, 6 to 12, or 6 to 10 carbon atoms. Non-limiting examples of aryl include, but are not limited to, phenyl, biphenyl, terphenyl, anthracenyl, naphthyl, acenaphthenyl, anthraquinonyl, indenyl, isoindenyl, indanyl, phenanthrenyl, anthracenyl, pyrenyl, and fluorenyl. The aryl may be optionally substituted with halogen, C₁₋₆ alkyl, C₃₋₁₀ cycloalkyl, or =O. In some embodiments, the aryl includes phenyl, naphthyl, anthracenyl, etc.

As used herein, the term "arylene" refers to a divalent group formed by the removal of a hydrogen atom from the aromatic ring moiety of the aryl as described above. The arylene may have 6-20 carbon atoms. In some embodiments, the arylene contains 6 to 18, 6 to 16, 6 to 14, 6 to 12, or 6 to 10 carbon atoms. In some embodiments, the aryl includes phenylene, naphthylene, etc.

As used herein, the term "amine group" refers to a group formed by the substitution of a hydrogen in amino with one or two hydrocarbyl. The amine group includes a secondary amine group (-NHR^{a}) or a tertiary amine group (-NR^{a}R^{b}, wherein R^{a} and R^{b} may be identical hydrocarbyl or different hydrocarbyl). Non-limiting examples of amine groups include, but are not limited to, a methylamine group, a dimethylamine group, an ethylamine group, a diethylamine group, a 2-propylamine group, a 1-propylamine group, a di(n-propyl)amine group, a di(isopropyl)amine group, a methyl n-propylamine group, a tert-butylamine group, etc. Herein, the term "amino" is used interchangeably with "primary amine group".

As used herein, the term "halogen" or "halo" refers to chlorine, bromine, fluorine, or iodine. The term "acyl" refers to an alkylcarbonyl or arylcarbonyl substituent. The term "acylamino" refers to an amide group linked to a nitrogen atom (i.e., R-CO-NH-). The term "carbamoyl" refers to an amide group linked to a carbonyl carbon atom (i.e., NH₂-CO-). The nitrogen atom on the acylamino or carbamoyl substituent is further optionally substituted. The term "sulfonamido" refers to a sulfonamide substituent linked via a sulfur or nitrogen atom. The term "amino" refers to a -NH₂ group. The term "hydroxyl" refers to an -OH group. The term "carboxyl" refers to a -CO₂H group.

Herein, the number of carbon atoms in a hydrocarbyl group, e.g., alkyl, alkenyl, cycloalkyl, cycloalkenyl, alkynyl, and aryl, is indicated by the prefix "Cₓ-C_{y} or C_{x-y}", wherein x is the minimum number of carbon atoms in the group and y is the maximum number of carbon atoms in the group. For example, "C₁-C₄ alkyl" or "C₁₋₄ alkyl" refers to an alkyl group containing 1 to 4 carbon atoms.

In the present invention, an azido-containing 2-picolinaldehyde derivative, as a N-terminal labeling probe (NLP), will bind to the nitrogen terminus of a protein to mediate the subsequent conjugation reaction with a nucleic acid molecule.

The azido-containing 2-picolinaldehyde derivative has a structural formula as shown below: 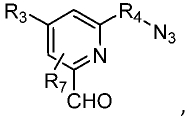 wherein R₃ is selected from hydrogen, alkyl, alkoxy, hydroxyl, amino, amine group; R₄ is selected from alkylene, arylene, PEG (polyethylene glycol repeat unit, i.e., -[OCH₂CH₂]ₘ-, wherein m may be an integer of 1-20, 1-16, 1-12, 1-8, 1-6, 1-4); R₇ is selected from hydrogen, alkyl, alkoxy.

R₇ is optionally substituted at positions 3 and 5 of the pyridine ring. Optionally, the azido-containing 2-picolinaldehyde derivative may have 1 or 2 identical or different R₇ groups.

In some embodiments, the alkyl from which R₃ is selected is, for example, C₁-C₈ alkyl, C₁-C₆ alkyl, C₁-C₄ alkyl, preferably methyl, ethyl, propyl, butyl. The alkoxy from which R₃ is selected is, for example, C₁-C₈ alkoxy, C₁-C₆alkoxy, C₁-C₄ alkoxy, preferably methoxy, ethoxy, propoxy, butoxy.

In some embodiments, the alkylene from which R₄ is selected is, for example, C₁-C₈ alkylene, C₁-C₆ alkylene, C₁-C₄ alkylene, preferably methylene, ethylene, propylene, butylene. The arylene from which R₄ is selected is, for example, C₆-C₁₄ arylene, C₆-C₁₂ arylene, C₆-C₁₀ arylene, preferably phenylene.

In some embodiments, the alkyl from which R₇ is selected is, for example, C₁-C₈ alkyl, C₁-C₆ alkyl, C₁-C₄ alkyl, preferably methyl, ethyl, propyl, butyl. The alkoxy from which R₇ is selected is, for example, C₁-C₈ alkoxy, C₁-C₆alkoxy, C₁-C₄ alkoxy, preferably methoxy, ethoxy, propoxy, butoxy.

In some embodiments, R₃, R₄, and R₇ are each optionally further substituted.

In some embodiments, the azido-containing 2-picolinaldehyde derivative is a 6-(azidomethyl)-2-picolinaldehyde derivative.

Herein, the 6-(azidomethyl)-2-picolinaldehyde derivative includes 6-(azidomethyl)-2-picolinaldehyde and derivatives having one to more substituents on the pyridine ring. In some embodiments, the substituent is preferably alkoxy, e.g., C₁-C₈ alkoxy, C₁-C₆ alkoxy, C₁-C₄ alkoxy, specifically methoxy, ethoxy, etc.

In some embodiments, the 6-(azidomethyl)-2-picolinaldehyde derivative is selected from

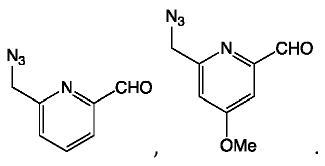

The synthesis of the 6-(azidomethyl)-2-picolinaldehyde derivative can be performed by a person skilled in the art according to known methods. For example, an azido-containing 2-picolinaldehyde derivative is obtained by reacting a bromo-substituted 2-picolinaldehyde compound with an azide. Specifically, in some embodiments, 6-(bromomethyl)-4-methoxypicolinaldehyde reacts with sodium azide in a solvent such as DMF for several hours by heating to obtain 6-(azidomethyl)-4-methoxypicolinaldehyde for use as NLP.

In the present invention, the maleimide derivative has the structure of: 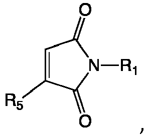 wherein R₁ is selected from hydrogen, hydrocarbyl, amine group, amino, hydroxyl, carboxyl, and the hydrocarbyl optionally has one or more substituents selected from hydrocarbyl, amine group, amino, hydroxyl, carboxyl. The hydrocarbyl is preferably alkyl, aryl, or a combination thereof, wherein the alkyl optionally has one or more identical or different substituents selected from aryl, amine group, amino, hydroxyl, carboxyl, the aryl optionally has one or more identical or different substituents selected from alkyl, amine group, amino, hydroxyl, carboxyl, or R₁ is a fluorophore, a PEG modification group, a biotin modification group, a polypeptide modification group, a nucleic acid modification group; R₅ is selected from hydrogen and alkyl.

In some embodiments, the hydrocarbyl from which R₁ is selected is, for example, C₁-C₈ alkyl, C₁-C₆ alkyl, C₁-C₄ alkyl, preferably methyl, ethyl, propyl, butyl. The alkyl may further have one or more identical or different substituents selected from C₆-C₁₄ aryl, amine group, amino, hydroxyl, carboxyl.

In some embodiments, the hydrocarbyl from which R₁ is selected is, for example, C₆-C₁₄ aryl, C₆-C₁₂ aryl, C₆-C₁₀ aryl, preferably phenyl, naphthyl. The aryl may further have one or more identical or different substituents selected from C₁-C₈ alkyl, amine group, amino, hydroxyl, carboxyl.

In some embodiments, the alkyl from which R₅ is selected is, for example, C₁-C₈ alkyl, C₁-C₆ alkyl, C₁-C₄ alkyl, preferably methyl, ethyl, propyl, butyl.

In some embodiments, R₁ and R₅ are each optionally further substituted.

In some embodiment, examples of maleimide derivatives that may be enumerated include the following compounds, but the present invention is not limited thereto.

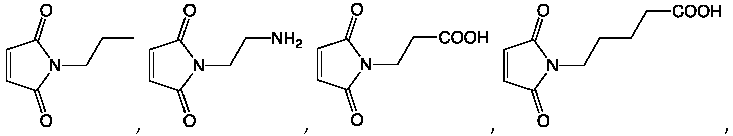

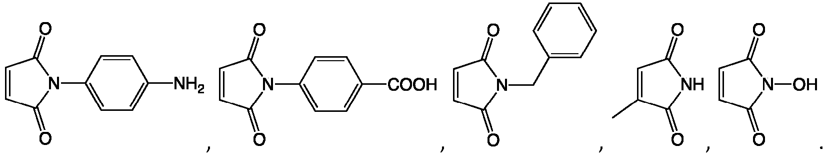

The modified protein, the azido-containing 2-picolinaldehyde derivative, and the maleimide derivative react in a molar ratio of 1 : 5-60 : 5-40, and the reaction continues overnight to obtain the protein moiety-linker conjugate of the present invention. In some embodiments, it is preferred that the molar ratio of the reactants is 1 : 10 : 5.

In some embodiments, the molar ratio of the azido-containing 2-picolinaldehyde derivative to the maleimide derivative is in the range of 30 : 1 to 1 : 1, e.g., 30 : 1, 20 : 1, 15 : 1, 12 : 1, 10 : 1, 8 : 1, 6 : 1, 5 : 1, 4 : 1, 3 : 1, 2 : 1, 1.5 : 1, 1: 1, preferably 4 : 1 to 1 : 1, more preferably 2 : 1.

In some embodiments, the reaction of the modified protein with the azido-containing 2-picolinaldehyde derivative and the maleimide derivative is performed in a buffer system. Buffer systems that can be suitable for use in the present invention include, but are not limited to, a BES buffer system, a Bis-Tris buffer system, an HEPES buffer system, a MOPS buffer system, a Phos buffer system, a TAPS buffer system, a Tris buffer system, etc. In some embodiments, the buffer system is preferably a phosphate buffer system (Phos buffer).

In some embodiments, the reaction of the modified protein with the azido-containing 2-picolinaldehyde derivative and the maleimide derivative is performed at pH 6.0-9.0, e.g., pH 6.0, 6.5, 7.0, 7.5, 8.0, 8.5, 9.0; pH 6.5-8.5, pH 7.0-8.0; pH 7.1, 7.2, 7.3, 7.4, 7.5, 7.6, 7.7, 7.8, 7.9, 8.0; preferably pH 7.2-7.8, more preferably pH 7.4, 7.5, 7.6.

Thus, the present inventors have successfully obtained a novel protein moiety-linker conjugate having a structure as shown in formula (I): 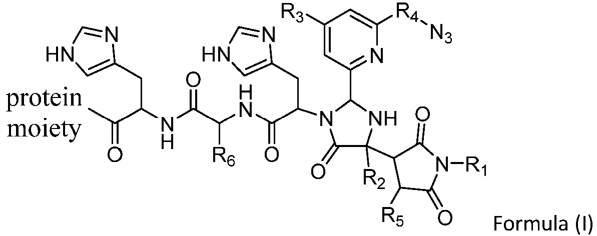
wherein R₁ is selected from hydrogen, hydrocarbyl, amine group, amino, hydroxyl, carboxyl, and the hydrocarbyl optionally has one or more substituents selected from hydrocarbyl, amine group, amino, hydroxyl, carboxyl. The hydrocarbyl is preferably alkyl, aryl, or a combination thereof, wherein the alkyl optionally has one or more identical or different substituents selected from aryl, amine group, amino, hydroxyl, carboxyl, the aryl optionally has one or more identical or different substituents selected from alkyl, amine group, amino, hydroxyl, carboxyl, or R₁ is selected from a fluorophore, a PEG modification group, a biotin modification group, a polypeptide modification group, a nucleic acid modification group;
R₂ is selected from a side chain moiety of any one of natural amino acids;
R₃ is selected from hydrogen, alkyl, alkoxy, hydroxyl, amino, amine group;
R₄ is selected from alkylene, arylene, PEG;
R₅ is selected from hydrogen and alkyl;
R₆ is selected from a side chain moiety of any one of natural amino acids.

In some embodiments, the hydrocarbyl from which R₁ is selected is, for example, C₁-C₈ alkyl, C₁-C₆ alkyl, C₁-C₄ alkyl, preferably methyl, ethyl, propyl, butyl. The alkyl may further have one or more identical or different substituents selected from C₆-C₁₄ aryl, amine group, amino, hydroxyl, carboxyl.

In some embodiments, the hydrocarbyl from which R₁ is selected is, for example, C₆-C₁₄aryl, C₆-C₁₂ aryl, C₆-C₁₀ aryl, preferably phenyl, naphthyl. The aryl may further have one or more identical or different substituents selected from C₁-C₈ alkyl, amine group, amino, hydroxyl, carboxyl.

In a preferred embodiment, R₁ is selected from propyl, ethylamine group, propionic acid group, valeric acid group, anilino group, benzoic acid group, tolyl, hydroxyl.

In a preferred embodiment, R₂ is selected from a side chain moiety of methionine.

In some embodiments, the alkyl from which R₃ is selected is, for example, C₁-C₈ alkyl, C₁-C₆ alkyl, C₁-C₄ alkyl, preferably methyl, ethyl, propyl, butyl. The alkoxy from which R₃ is selected is, for example, C₁-C₈ alkoxy, C₁-C₆ alkoxy, C₁-C₄ alkoxy, preferably methoxy, ethoxy, propoxy, butoxy.

In a preferred embodiment, R₃ is selected from methoxy.

In some embodiments, the alkylene from which R₄ is selected is, for example, C₁-C₈ alkylene, C₁-C₆ alkylene, C₁-C₄ alkylene, preferably methylene, ethylene, propylene, butylene. The arylene from which R₄ is selected is, for example, C₆-C₁₄ arylene, C₆-C₁₂ arylene, C₆-C₁₀ arylene, preferably phenylene.

In a preferred embodiment, R₄ is selected from methylene.

In some embodiments, the alkyl from which R₅ is selected is, for example, C₁-C₈ alkyl, C₁-C₆ alkyl, C₁-C₄ alkyl, preferably methyl, ethyl, propyl, butyl.

In a preferred embodiment, R₅ is selected from methyl.

In a preferred embodiment, R₆ is selected from a side chain moiety of histidine.

In some embodiments, R₁, R₃, R₄ and R₅ are each optionally further substituted.

In a preferred embodiment, n is an integer selected from 1-8, e.g., 1, 2, 3, 4, 5, 6, 7, 8.

In a preferred embodiment, the protein moiety-linker conjugate has a structure as shown below:

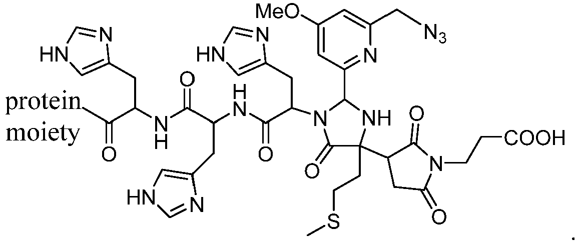

In some embodiments, R₁ of the protein moiety-linker conjugate of the present invention is a biologically active molecule moiety selected from a fluorophore, a PEG modification group, a biotin modification group, a polypeptide modification group, a nucleic acid modification group, and thus can be suitable for use in a variety of biological, chemical, medical, pharmaceutical applications. For example, the fluorophore includes, but is not limited to, common fluorophores such as FAM, ROX, and CY5; the PEG modification group includes, but is not limited to, PEG4000, PEG5000, etc.; the polypeptide modification group is, for example, GRKKRRQRRRPQ, etc.; the small drug molecule is, for example, MMAE (monomethyl auristatin E), DM1 (myotonic dystrophy type 1), etc.

In one aspect, the protein moiety-linker conjugate of the present invention is further subjected to a linking reaction with an active molecule via azido, thereby forming a protein-active molecule conjugate. In the present invention, the active molecule includes, but is not limited to, a nucleic acid molecule, a fluorescent molecule, a PEG molecule, a biotin molecule, a polypeptide molecule, a small drug molecule, etc. Thus, the protein-active molecule conjugate obtained after linking to the protein moiety-linker conjugate may comprise the following groups, e.g., a fluorophore including, but not limited to, common fluorophores such as FAM, ROX, and CY5; a PEG modification group including, but not limited to, PEG4000, PEG5000, etc.; a polypeptide modification group, such as GRKKRRQRRRPQ; a small drug molecule, such as MMAE (monomethyl auristatin E), DM1 (myotonic dystrophy type 1).

In the present invention, the linking of the protein moiety-linker conjugate to the active molecule is performed via a reaction between azido and alkynyl. Therefore, the active molecule needs to be modified prior to conjugation with the protein moiety-linker conjugate. In some embodiments, the active molecule is modified with an alkynyl-containing compound. As used herein, the term "alkynyl-containing compound" refers to a compound containing one or more carbon-carbon triple bonds. The alkynyl-containing compound includes, but is not limited to, a linear alkyne compound, a substituted linear alkyne compound, a cycloalkyne compound, a substituted cycloalkyne compound, etc. The active molecule is modified with an alkynyl-containing compound to have an alkynyl substituent containing one or more carbon-carbon triple bonds.

In a preferred embodiment, the alkynyl-containing compound is preferably dibenzocyclooctyne carboxylic acid.

In some embodiments, the active molecule is a nucleic acid molecule.

In a preferred embodiment, the nucleic acid is modified to have a substituent selected from dibenzocyclooctynyl, bicyclo[6.1.0]nonynyl, etc.

In a preferred embodiment, the dibenzocyclooctyne carboxylic acid is used to obtain a dibenzocyclooctyne-modified nucleic acid.

Nucleic acids that can be used in the method of the present invention are not particularly limited. As used herein, the term "nucleic acid" encompasses any type of nucleic acid and refers to any polymer of nucleotides (e.g., deoxyribonucleotides, ribonucleotides) of any length containing purine and pyrimidine bases, or other natural, chemically or biochemically modified, unnatural, or derived nucleotide bases. The term "unnatural nucleotide" or "modified nucleotide" refers to a nucleotide that contains a modified nitrogenous base, sugar, or phosphate group, or that incorporates an unnatural moiety in its structure. Examples of unnatural nucleotides include dideoxynucleotides, and biotinylated, aminated, deaminated, alkylated, benzylated, and fluorescently labeled nucleotides. The term "nucleic acid" includes DNA and RNA, e.g., but not limited to, genomic or chromosomal DNA, plasmid DNA, amplified DNA, cDNA, total RNA, mRNA, small RNA, etc. The term "nucleic acid" is intended to include natural DNA and/or RNA molecules, or synthetic DNA and/or RNA molecules. In the present invention, the backbone of the nucleic acid may contain sugar and phosphate groups commonly found in RNA or DNA, or modified or substituted sugar or phosphate groups. The nucleic acid may contain modified nucleotides, e.g., methylated nucleotides and nucleotide analogs, e.g., base modifications such as 2'-F modification, 2'-OMe modification, or 2'-MOE modification. The nucleic acid may also have other modifications, including, but not limited to, a biotin modification, a fluorophore modification, a polypeptide modification, a PEG modification, a phosphorylation modification, etc.

In some embodiments, the nucleic acid can be obtained by isolation and purification from cells containing the nucleic acid from a natural source. In some embodiments, the nucleic acid can be obtained by chemical synthesis or other known protocols for the production of nucleic acids. In some embodiments, the nucleic acid has a length of 10 to 100 nt, e.g., a length of 10 to 90 nt, 10 to 80 nt, 10 to 70 nt, 10 to 60 nt, 10 to 50 nt, 10 to 40 nt, 10 to 30 nt, 10 to 20 nt, e.g., a length of 10 nt, 20 nt, 30 nt, 40 nt, 50 nt, 60 nt, 70 nt, 80 nt, 90 nt, 100 nt, e.g., a length of any integer number of nt between 10 and 100 nt.

The modification reaction of the nucleic acid can be performed by a person skilled in the art according to well-known methods. The amino-modified nucleic acid reacts with an alkynyl-containing compound.

In the present invention, the position of an amino modification may be the 3' end, the 5' end, or any position between the two ends of the nucleic acid. The amino-modified nucleic acid can be synthesized by a person skilled in the art according to well-known methods, and commercially available amino-modified nucleic acid products are also available.

In some embodiments, an amino-modified nucleic acid reacts with an alkynyl-containing carboxylic acid compound having the following structural formula in the presence of an amine to obtain a nucleic acid with an alkynyl-containing group modification: 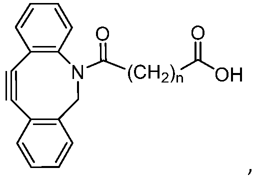 wherein n = 1-8, e.g., 1-6, 1-4, such as 1, 2, 3, and 4. The choice of amine can be determined by a person skilled in the art according to common general knowledge, as long as it can catalyze the linking reaction between carboxyl and amino.

In a preferred embodiment, the nucleic acid with 3'-end amino modification reacts with dibenzocyclooctyne carboxylic acid having the following structural formula in the presence of an amine: 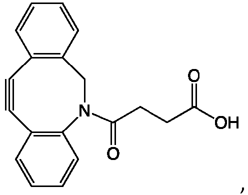 dibenzocyclooctyne carboxylic acid.

In the above reaction, the nucleic acid reacts with the alkynyl-containing carboxylic acid compound in a molar ratio of 200 : 1-20 : 1, e.g., 200 : 1, 150 : 1, 100 : 1, 80 : 1, 60 : 1, 40 : 1, 20 : 1.

In the present invention, when the amino modification is at the 3' end or the 5' end of a nucleic acid, the corresponding other end may be unmodified or may be modified with common modification groups in the art, such as biotin and FLAG TAG, to obtain corresponding biological properties. When the amino modification is at any position between the two ends of a nucleic acid, both ends may be each independently unmodified or modified with common modification groups in the art, such as biotin and FLAG TAG, to obtain corresponding biological properties.

After the above reaction, the nucleic acid with alkynyl-containing group modification at the 3' end, the 5' end, or any position between the two ends is obtained.

After obtaining the active molecule with alkynyl-containing group modification, it reacts with the protein moiety-linker conjugate of the present invention, for example, overnight at room temperature, to obtain a protein-active molecule conjugate.

In some embodiments, the reaction of the protein moiety-linker conjugate with the active molecule with alkynyl-containing group modification is performed in a buffer system. Buffer systems that can be suitable for use in the present invention include, but are not limited to, a BES buffer system, a Bis-Tris buffer system, an HEPES buffer system, a MOPS buffer system, a Phos buffer system, a TAPS buffer system, a Tris buffer system, etc. In some embodiments, the buffer system is preferably a phosphate buffer system (Phos buffer).

In some embodiments, the reaction of the protein moiety-linker conjugate with the active molecule with alkynyl group-containing group modification is performed at pH 6.0-9.0, e.g., pH 6.0, 6.5, 7.0, 7.5, 8.0, 8.5, 9.0; pH 6.5-8.5, pH 7.0-8.0; pH 7.1, 7.2, 7.3, 7.4, 7.5, 7.6, 7.7, 7.8, 7.9, 8.0; preferably pH 7.2-7.8, more preferably pH 7.4, 7.5, 7.6.

In some embodiments, the active molecule is a nucleic acid molecule, and the nucleic acid molecule with alkynyl-containing group modification has a structure as shown in the following formula: 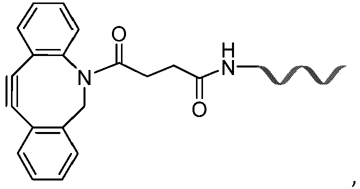 wherein 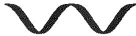 represents the nucleic acid moiety.

Thus, in some embodiments, the protein moiety-linker conjugate is linked to the nucleic acid molecule with alkynyl-containing group modification to obtain a protein-nucleic acid conjugate of the following structural formula: 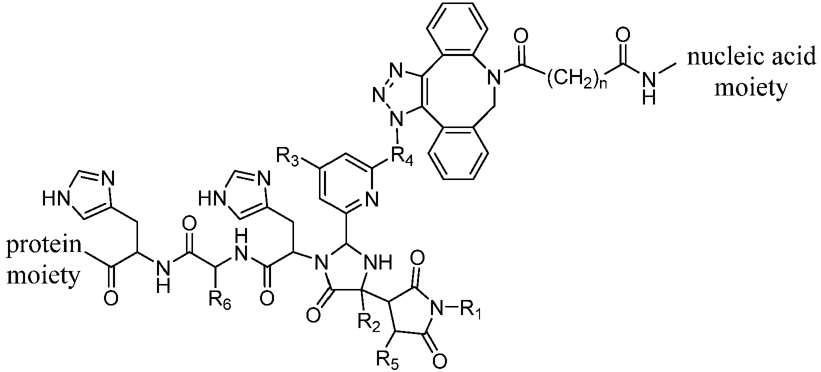 wherein R₁, R₂, R₃, R₄, R₅, R₆, and n are as defined above.

In a preferred embodiment, the protein-nucleic acid conjugate has the structure of:

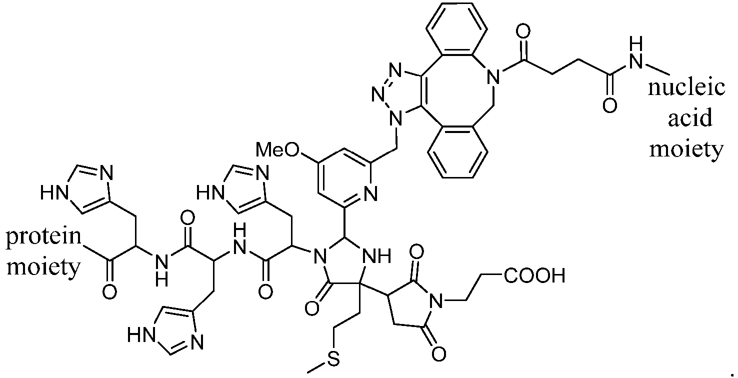

### Examples

The technical solutions of the present invention are further illustrated in more detail by the examples and in conjunction with the accompanying drawings. Unless otherwise specified, the reagents used in the following examples are all conventional reagents in the art, which are commercially available or formulated according to conventional methods in the art, with the specification of laboratory reagents; the experimental methods and experimental conditions used are those conventional in the art and can be referred to the relevant laboratory manuals, well-known literature, or manufacturer's instructions.

### Instrument

PCR instrument model A37834, purchased from ThermoFisher.

HPLC: LC-20AD, purchased from Shimadzu.

LC-MS: 1260 Infinity II HPLC and 6230 TOF, purchased from Agilent.

### Example 1. Preparation of azido-containing 2-picolinaldehyde derivative (NLP)

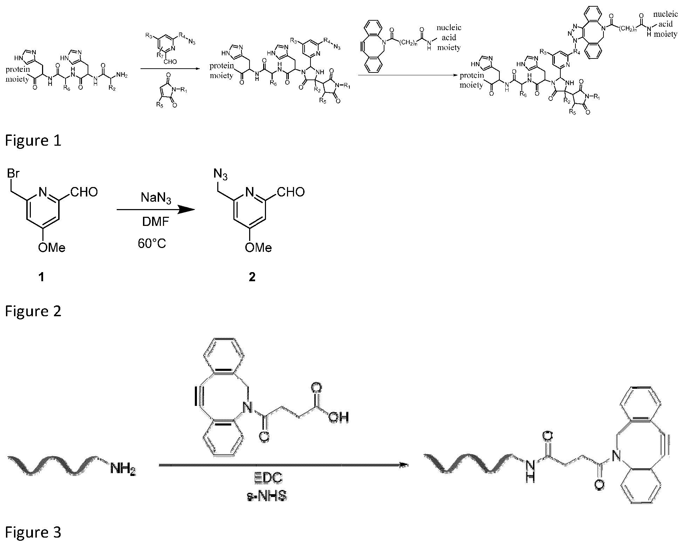

The reaction is as shown in FIG. 2.

Experimental steps: Bromo compound 1 (11.5 mg, 50 µmol) was dissolved in 80 µL of DMF solution, NaN₃ (6.5 mg, 100 µmol) was added, and a reaction was performed under heating at 60°C for 16 hours. 500 µL of water was added to the reaction liquid, and extraction was performed 3 times with ethyl acetate. The organic layers were combined and concentrated under reduced pressure to give a crude product of compound 2, i.e., 6-(azidomethyl)-4-methoxypicolinaldehyde, as a dark yellow liquid. The crude product was used without further purification.

### Example 2. Preparation of dibenzocyclooctyne-modified nucleic acid

The reaction is as shown in FIG. 3.

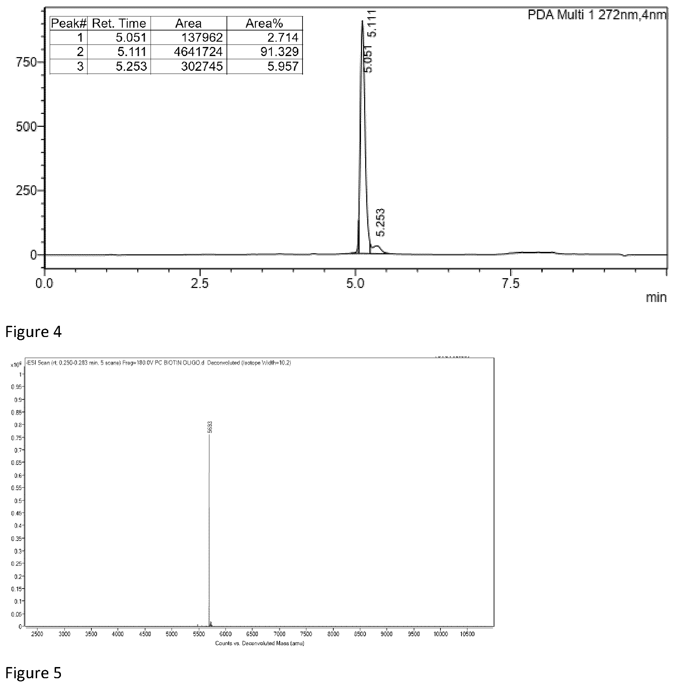

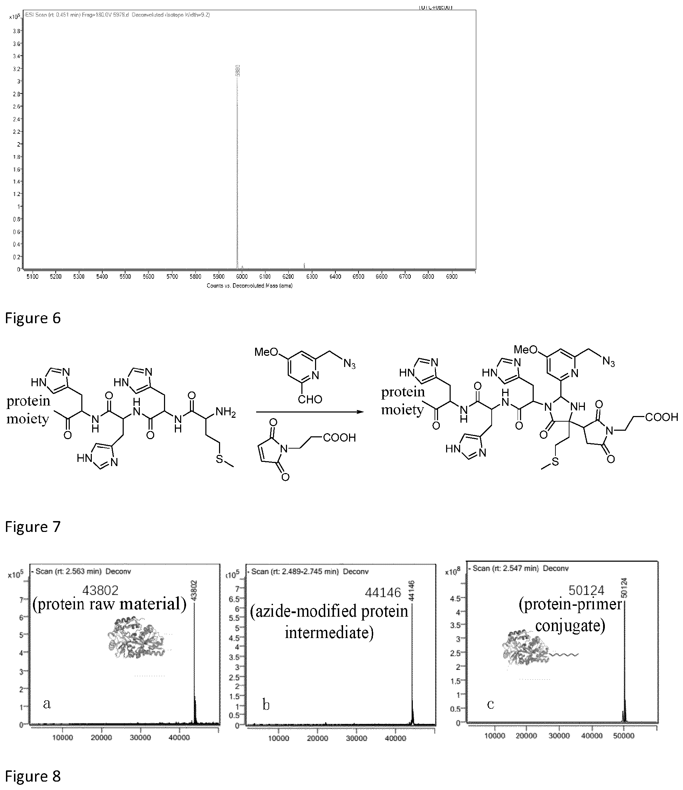

Experimental steps: 6.1 mg of dibenzocyclooctyne carboxylic acid (DBCO acid) was dissolved in 160 µL of DMSO solution, 4 mg of EDC (1-(3-dimethylaminopropyl)-3-ethylcarbodiimide) and 4 mg of s-NHS (thiosuccinimide) were added, and activation was performed at room temperature for 30 minutes. At the end of the activation, 200 nmol nucleic acid with 3'-end amino modification and 5'-end biotin modification (dissolved in 500 µL of phosphate buffer with pH 7.54) and 340 µL of DMSO were added, and the resulting mixture was placed in a shaking mixer and shaken for a reaction overnight at 1200 rpm at room temperature. After the sedimentation was completed, a crude sample of dibenzocyclooctyne-modified nucleic acid was obtained. The crude sample was taken and subjected to HPLC purification by gradient HPLC using 10%-80% acetonitrile phase over 20 minutes, and the purified sample was lyophilized. After the lyophilization, a pure product of dibenzocyclooctyne-modified nucleic acid was obtained. 0.5 nmol purified nucleic acid was taken separately for characterizations by HPLC and LC-MS, in which the HPLC analysis gradient was: 5%-50% acetonitrile phase, 6 minutes; the LC-MS gradient was: 5%-80% acetonitrile phase, 1.5 minutes. The HPLC analysis profile is as shown in FIG. 4. The mass spectrometry profiles are as shown in FIG. 5 and FIG. 6.

Sequence of nucleic acid with 5'-end biotin and 3'-end amino:
<PCbt>ACAACATATAGCGCAG<3am> (SEQ ID NO. 1)
where <PCbt> is a 5'-end photocleavable biotin modification, and <3am> is a 3'-end amino modification (Genscript)

**Table 1. Molecular weights of test samples detected by mass spectrometry**

| Test sample | Detected molecular weight | Theoretical molecular weight |
|---|---|---|
| Oligonucleotide with 3'-end amino modification and 5'-end biotin modification | 5693 | 5692 |
| Oligonucleotide with 3'-end DBCO modification and 5'-end biotin modification | 5978 | 5979 |

Conclusion: Using the conditions provided, a dibenzocyclooctyne (DBCO)-modified nucleic acid can be obtained.

### Example 3. Preparation of protein moiety-linker conjugate

In this example, the protein moiety-linker conjugate of the present invention was prepared from different proteins by using the preparation method of the present invention. The proteins used in this example included a maltose-binding protein, a green fluorescent protein, a ubiquitin protein, and a small ubiquitin-like modifier protein. The amino acid sequences of proteins with a N-terminal His tag modification are as follows:
H6-MBP: (SEQ ID NO. 2) 
MHHHHHHKIEEGKLVIWINGDKGYNGLAEVGKKFEKDTGIKVTVEHPDKLEEKFPQVAATGDGPDIIFWAHD
RFGGYAQSGLLAEITPDKAFQDKLYPFTWDAVRYNGKLIAYPIAVEALSLIYNKDLLPNPPKTWEEIPALDKELK
AKGKSALMFNLQEPYFTWPLIAADGGYAFKYENGKYDIKDVGVDNAGAKAGLTFLVDLIKNKHMNADTDYSI
AEAAFNKGETAMTINGPWAWSNIDTSKVNYGVTVLPTFKGQPSKPFVGVLSAGINAASPNKELAKEFLENYLL
TDEGLEAVNKDKPLGAVALKSYEEELVKDPRIAATMENAQKGEIMPNIPQMSAFWYAVRTAVINAASGRQT
VDEALKDAQTNSSSNNNNNNNNNNLGIEGRISHM (Genscript)
H6-GFP: (SEQ ID NO. 3) 
MHHHHHHVSKGEELFTGVVPILVELDGDVNGHKFSVSGEGEGDATYGKLTLKFICTTGKLPVPWPTLVTTLTY
GVQCFSRYPDHMKQHDFFKSAMPEGYVQERTIFFKDDGNYKTRAEVKFEGDTLVNRIELKGIDFKEDGNILG
HKLEYNYNSHNVYIMADKQKNGIKVNFKIRHNIEDGSVQLADHYQQNTPIGDGPVLLPDNHYLSTQSALSKD
PNEKRDHMVLLEFVTAAGITLGMDELYK (Genscript)
H6-Ubiquitin: (SEQ ID NO. 4) 
MHHHHHHQIFVKTLTGKTITLEVEPSDTIENVKAKIQDKEGIPPDQQRLIFAGKQLEDGRTLSDYNIQKESTLHL
VLRLRGG (Genscript)
H6-SUMO: (SEQ ID NO. 5)

MHHHHHHMSDSEVNQEAKPEVKPEVKPETHINLKVSDGSSEIFFKIKKTTPLRRLMEAFAKRQGKEMDSLRFL
Y DGIRIQADQTPEDLDMEDNDIIEAHREQIGGATY (Genscript)

The reaction is as shown in FIG. 7.

### Experimental steps:

Step 1: 3 nmol protein was added to 30 µL of 50 mM phosphate buffer solution with pH 7.4, 30 nmol compound 2 (NLP, dissolved in 1 µL of DMSO) obtained in Example 1 and 15 nmol 3-maleimidopropionic acid (dissolved in 1 µL of DMSO) were added, and a reaction was performed at 37°C overnight in a PCR instrument;

Step 2: The reaction liquid was transferred to a 0.5 mL ultrafiltration tube and diluted to 500 µL by adding a phosphate buffer, the sample was concentrated to 30 µL by centrifugation at 12000 rpm for 10 minutes, and this washing process was repeated three times to remove excess small molecule compound to obtain a protein moiety-linker conjugate.

Table 2 shows the molecular weights of reactant and product detected by mass spectrometry when maltose-binding protein is used.

**Table 2: Molecular weights of test samples detected by mass spectrometry**

| Test sample | Observed molecular weight | Theoretical molecular weight |
|---|---|---|
| Protein raw material (maltose-binding protein) | 43802 | 43801 |
| Protein moiety-linker conjugate | 44146 | 44145 |

Conclusion: Using the conditions provided, NLP and 3-maleimidopropionic acid are simultaneously added to the protein, and a protein moiety-linker conjugate can be obtained.

### Example 4. Synthesis of protein-nucleic acid conjugate

In this example, the protein moiety-linker conjugate of the present invention reacted with a modified nucleic acid to prepare a protein-nucleic acid conjugate.

### Experimental steps:

Step 3: 4.5 nmol nucleic acid with 3'-end DBCO modification and 5'-end biotin modification was added to the protein moiety-linker conjugate obtained in Example 3 dissolved in 30 µL of 50 mM phosphate buffer solution with pH 7.4, and a reaction was performed at room temperature overnight to obtain a protein-nucleic acid conjugate. When a maltose-binding protein is used, the molecular weight of the protein-nucleic acid conjugate detected by mass spectrometry is 50124, and the theoretical value is 50124.

Conclusion: Using the conditions provided, the protein moiety-linker conjugate of the present invention reacts with a modified nucleic acid to prepare a protein-nucleic acid conjugate.

The mass spectrometry profiles of the maltose-binding protein and various modification products thereof before, during, and after the reaction are as shown in FIG. 8.

### Example 5. Analysis and test of peptide fragment

In this example, the position of the modification site of the linker moiety on the protein was detected.

Experimental steps: The protein-nucleic acid conjugate sample was cleaved with trypsin, and the site, type and proportion of post-translational modifications that occurred in the analyzed sample were detected by liquid chromatography-mass spectrometry.

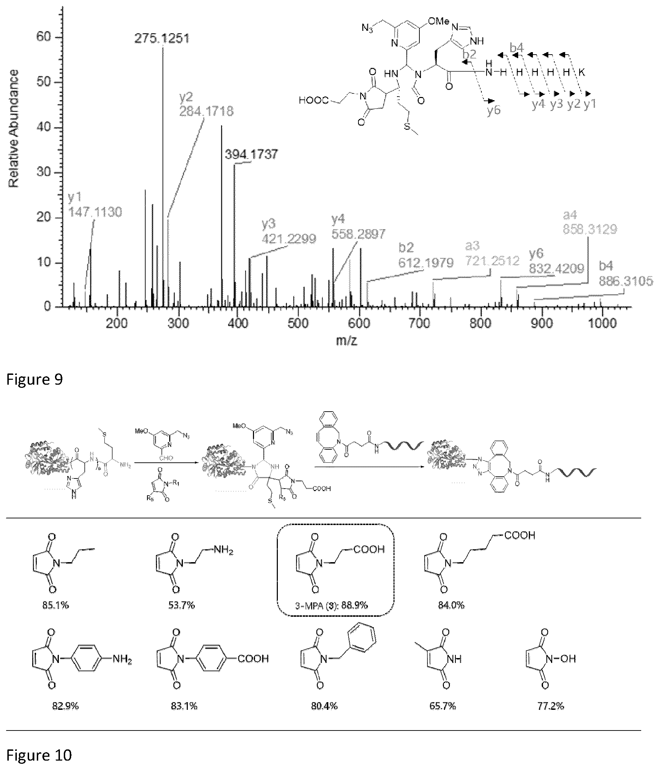

It can be seen from FIG. 9 that: when 6-(azidomethyl)-4-methoxypicolinaldehyde and 3-maleimidopropionic acid are used to modify the maltose-binding protein, the modification sites are located at the last two amino acids at the N-terminus. This also confirms that the azido-containing 2-picolinaldehyde derivative and the maleimide derivative together form an imidazolinone ring structure with the last two amino acid residues of the short peptide containing His tag attached to the N-terminus of the protein according to the method of the present invention.

### Example 6. Screening of maleimide derivative small molecules

In this example, the conversion rates of the synthesis reaction of the present invention under the mediation of different maleimide derivative small molecules were tested. The maleimide derivative small molecules used in this example included

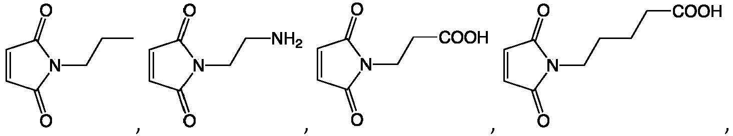

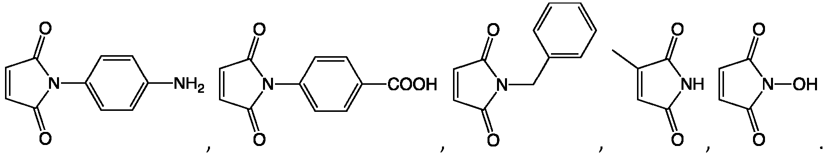

The reaction is as shown in FIG. 10. The reaction steps were the same as those described in Examples 3 and 4.

The protein used in this example was a maltose-binding protein, the short peptide containing His tag was Met(His)₆, the azido-containing 2-picolinaldehyde derivative was 6-(azidomethyl)-4-methoxypicolinaldehyde, and the modified nucleic acid was the dibenzocyclooctyne-modified nucleic acid prepared in Example 2.

Experimental steps:
Step 1: 3 nmol protein was added to 30 µL of 50 mM phosphate buffer solution with pH 7.4, 30 nmol compound 2 (NLP, dissolved in 1 µL of DMSO) obtained in Example 1 and 15 nmol maleimide derivative small molecule (dissolved in 1 µL of DMSO) were added, and a reaction was performed at 37°C overnight in a PCR instrument;
Step 2: The reaction liquid was transferred to a 0.5 mL ultrafiltration tube and diluted to 500 µL by adding a phosphate buffer, the sample was concentrated to 30 µL by centrifugation at 12000 rpm for 10 minutes, and this process was repeated three times to remove excess small molecule compound to obtain a protein moiety-linker conjugate;
Step 3: 4.5 nmol nucleic acid with 3'-end DBCO modification and 5'-end biotin modification was added to the protein moiety-linker conjugate dissolved in 30 µL of 50 mM phosphate buffer solution with pH 7.4, and a reaction was performed at room temperature overnight to obtain a protein-nucleic acid conjugate.

The proportion of the protein-nucleic acid conjugate was determined by SDS-PAGE gel. The results are as shown in FIG. 10.

### Conclusion:

The reaction of step 1 of the synthesis method of the present invention has good conversion rate under the mediation of a variety of maleimide derivative small molecules, among which the 3-maleimidopropionic acid results in the highest conversion rate.

### Example 7. Optimization of pH of reaction solution

In this example, the conversion rates of the synthesis reaction of the present invention at pH values were tested.

The reaction steps of this example were the same as those of Example 6, in which the protein used was a maltose-binding protein, the short peptide containing His tag was Met(His)₆, the azido-containing 2-picolinaldehyde derivative was 6-(azidomethyl)-4-methoxypicolinaldehyde, the maleimide derivative was 3-maleimidopropionic acid, and the modified nucleic acid was the dibenzocyclooctyne-modified nucleic acid prepared in Example 2.

Experimental steps:
Step 1: 3 nmol protein was added to 30 µL of 50 mM phosphate buffer solution at pH 6-9, 30 nmol compound 2 (NLP, dissolved in 1 µL of DMSO) obtained in Example 1 and 15 nmol 3-maleimidopropionic acid (dissolved in 1 µL of DMSO) were added, and a reaction was performed at 37°C overnight in a PCR instrument;
Step 2: The reaction liquid was transferred to a 0.5 mL ultrafiltration tube and diluted to 500 µL by adding a phosphate buffer, the sample was concentrated to 30 µL by centrifugation at 12000 rpm for 10 minutes, and this process was repeated three times to remove excess small molecule compound to obtain a protein moiety-linker conjugate;
Step 3: 4.5 nmol nucleic acid with 3'-end DBCO modification and 5'-end biotin modification was added to the protein moiety-linker conjugate dissolved in 30 µL of 50 mM phosphate buffer solution with pH 7.4, and a reaction was performed at room temperature overnight to obtain a protein-nucleic acid conjugate.

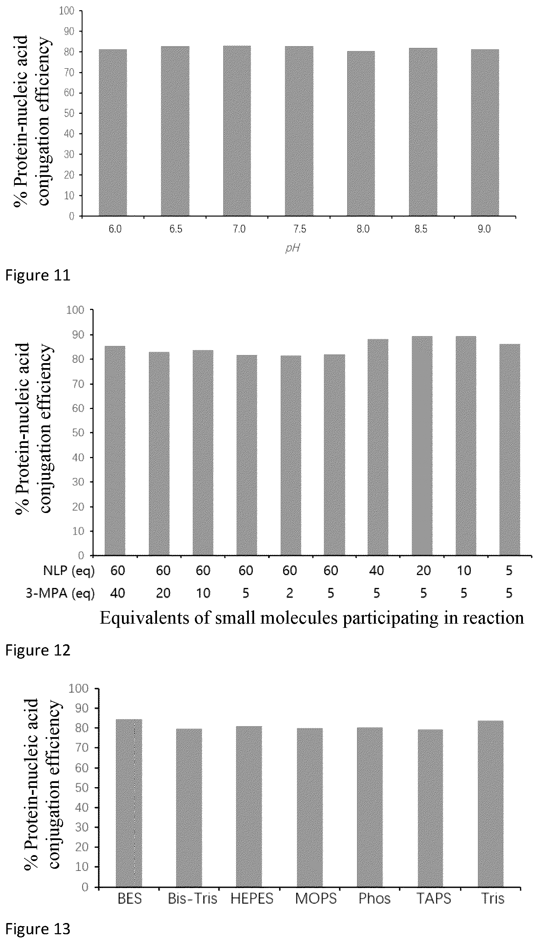

The proportion of the protein-nucleic acid conjugate was determined by SDS-PAGE gel. The results are as shown in FIG. 11.

### Conclusion:

The reaction of step 1 of the synthesis method of the present invention can be performed well in a buffer with a pH range of 6-9.

### Example 8. Optimization of equivalents of small molecules

In this example, the conversion rates of the synthesis reaction of the present invention with the participation of different equivalents of azido-containing 2-picolinaldehyde derivative small molecules and maleimide derivative small molecules were tested.

The reaction steps of this example were the same as those of Example 6, in which the protein used was a maltose-binding protein, the short peptide containing His tag was Met(His)₆, the azido-containing 2-picolinaldehyde derivative was 6-(azidomethyl)-4-methoxypicolinaldehyde, the maleimide derivative was 3-maleimidopropionic acid, and the modified nucleic acid was the dibenzocyclooctyne-modified nucleic acid prepared in Example 2.

### Experimental steps:

Step 1: 3 nmol protein was added to 30 µL of 50 mM phosphate buffer solution with pH 7.4, 30 nmol compound 2 (NLP, dissolved in 1 µL of DMSO) obtained in Example 1 and 15 nmol 3-maleimidopropionic acid (dissolved in 1 µL of DMSO) were added, and a reaction was performed at 37°C overnight in a PCR instrument.

Step 2: The reaction liquid was transferred to a 0.5 mL ultrafiltration tube and diluted to 500 µL by adding a phosphate buffer, the sample was concentrated to 30 µL by centrifugation at 12000 rpm for 10 minutes, and this process was repeated three times to remove excess small molecule compound to obtain a protein moiety-linker conjugate.

Step 3: 4.5 nmol nucleic acid with 3'-end DBCO modification and 5'-end biotin modification was added to the protein moiety-linker conjugate dissolved in 30 µL of 50 mM phosphate buffer solution with pH 7.4, and a reaction was performed at room temperature overnight to obtain a protein-nucleic acid conjugate.

The above reaction steps were repeated using different equivalents of NLP and 3-MPA (3-maleimidopropionic acid) as shown in Table 3. The proportion of the protein-nucleic acid conjugate was determined by SDS-PAGE gel. The results are as shown in FIG. 12.

**Table 3: Different equivalents of NLP (N-terminal labeling probe) and 3-MPA (3-maleimidopropionic acid)**

| | | | | | | | | | | |
|---|---|---|---|---|---|---|---|---|---|---|
| NLP (eq) | 60 | 60 | 60 | 60 | 60 | 60 | 40 | 20 | 10 | 5 |
| 3-MPA (eq) | 40 | 20 | 10 | 5 | 2 | 5 | 5 | 5 | 5 | 5 |

It can be seen from FIG. 12 that:
the reaction of step 1 of the synthesis method of the present invention has good conversion rates with the participation of different equivalents of small molecules.

### Example 9. Compatibility of reaction with different buffer salts

In this example, the compatibility of the synthesis reaction of the present invention in different buffer salts was tested. The buffer salt systems used in this example included BES, Bis-Tris, HEPES, MOPS, Phos, TAPS, or Tris.

The reaction steps of this example were the same as those of Example 6, in which the protein used was a maltose-binding protein, the short peptide containing His tag was Met(His)₆, the azido-containing 2-picolinaldehyde derivative was 6-(azidomethyl)-4-methoxypicolinaldehyde, the maleimide derivative was 3-maleimidopropionic acid, and the modified nucleic acid was the dibenzocyclooctyne-modified nucleic acid prepared in Example 2.

### Experimental steps:

Step 1: 3 nmol protein was added to 30 µL of 50 mM solutions of different buffer salts with pH 7.4, 30 nmol compound 2 (NLP, dissolved in 1 µL of DMSO) obtained in Example 1 and 15 nmol 3-maleimidopropionic acid (dissolved in 1 µL of DMSO) were added, and a reaction was performed at 37°C overnight in a PCR instrument.

Step 2: The reaction liquid was transferred to a 0.5 mL ultrafiltration tube and diluted to 500 µL by adding a phosphate buffer, the sample was concentrated to 30 µL by centrifugation at 12000 rpm for 10 minutes, and this process was repeated three times to remove excess small molecule compound to obtain a protein moiety-linker conjugate.

Step 3: 4.5 nmol nucleic acid with 3'-end DBCO modification and 5'-end biotin modification was added to the protein moiety-linker conjugate dissolved in 30 µL of 50 mM phosphate buffer solution with pH 7.4, and a reaction was performed at room temperature overnight to obtain a protein-nucleic acid conjugate. The proportion of the protein-nucleic acid conjugate was determined by SDS-PAGE gel. The results are as shown in FIG. 13.

### Conclusion:

The reaction of step 1 of the synthesis method of the present invention is compatible with a variety of buffer salt systems.

### Example 10. Compatibility of reaction with different additives

In this example, the compatibility of the synthesis reaction of the present invention with different additives added was tested. The additives used in this example included urea, guanidine hydrochloride, imidazole, 3-methylindole, phenol, and SDS. These additives mimic the side chain structure of common amino acids. The results of this example will confirm that the synthesis method of the present invention is highly specific and will only occur in short peptides containing His tag modified at the N-terminus of the protein. The amino acid side chains included in the protein do not affect the efficiency of the conjugation reaction of the present invention.

The reaction steps of this example were the same as those of Example 6, in which the protein used was a maltose-binding protein, the short peptide containing His tag was Met(His)₆, the azido-containing 2-picolinaldehyde derivative was 6-(azidomethyl)-4-methoxypicolinaldehyde, the maleimide derivative was 3-maleimidopropionic acid, and the modified nucleic acid was the dibenzocyclooctyne-modified nucleic acid prepared in Example 2.

### Experimental steps:

Step 1: 3 nmol protein was added to 30 µL of 50 mM phosphate buffer solution with pH 7.4, 5 mM small molecule additive was additionally added, 30 nmol compound 2 (NLP, dissolved in 1 µL of DMSO) obtained in Example 1 and 15 nmol 3-maleimidopropionic acid (dissolved in 1 µL of DMSO) were added, and a reaction was performed at 37°C overnight in a PCR instrument.

Step 2: The reaction liquid was transferred to a 0.5 mL ultrafiltration tube and diluted to 500 µL by adding a phosphate buffer, the sample was concentrated to 30 µL by centrifugation at 12000 rpm for 10 minutes, and this process was repeated three times to remove excess small molecule compound to obtain a protein moiety-linker conjugate.

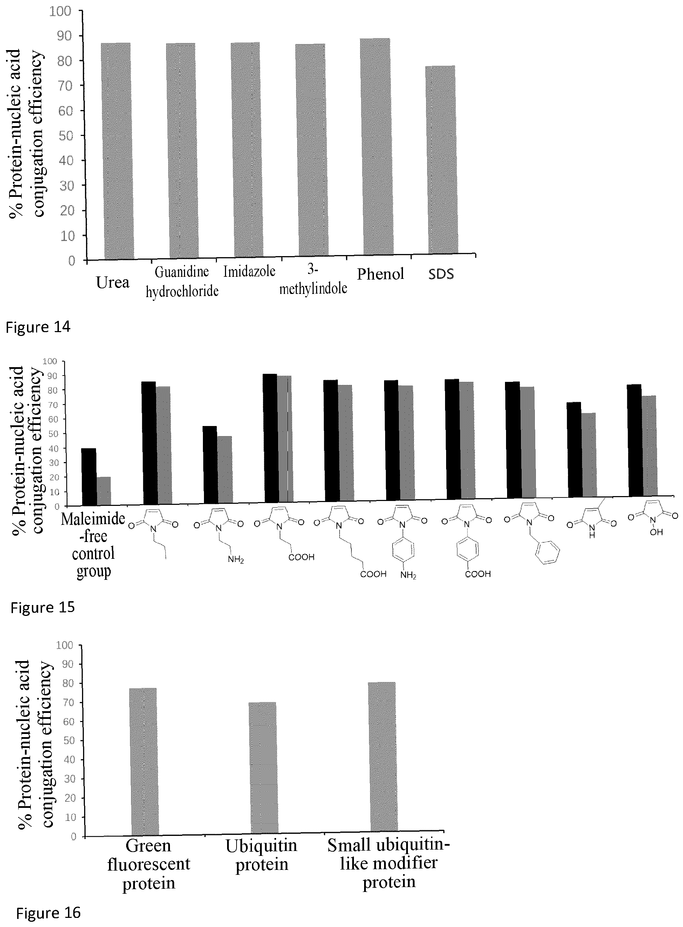

Step 3: 4.5 nmol nucleic acid with 3'-end DBCO modification and 5'-end biotin modification was added to the protein moiety-linker conjugate dissolved in 30 µL of 50 mM phosphate buffer solution with pH 7.4, and a reaction was performed at room temperature overnight to obtain a protein-nucleic acid conjugate. The proportion of the protein-nucleic acid conjugate was determined by SDS-PAGE gel. The results are as shown in FIG. 14.

### Conclusion:

The reaction of step 1 of the synthesis method of the present invention is compatible with a variety of small molecule additives commonly used in biology, and these small molecule additives do not affect the efficiency of the conjugation reaction of the present invention, indicating that the amino acid side chains included in the protein do not affect the efficiency of the conjugation reaction of the present invention.

### Example 11. Test of thermal stability of protein-nucleic acid conjugates

In this example, the effect of the addition of a maleimide derivative on the thermal stability of the protein-nucleic acid conjugate was tested.

### Experimental steps:

Following steps similar to those of Example 6, a protein-nucleic acid conjugate was obtained without the addition of a maleimide derivative, in which the intermediate had the structure of:

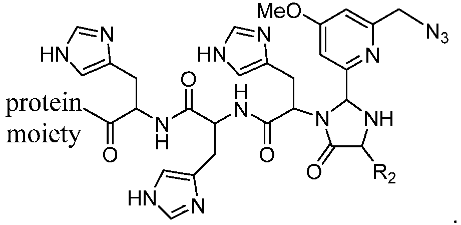

The protein-nucleic acid conjugate obtained without the addition of a maleimide derivative and the protein-nucleic acid conjugates obtained using different maleimide derivative small molecules according to Example 6 were separately heated in a PCR instrument at 95°C for 10 minutes. Subsequently, the proportions of the protein-nucleic acid conjugates before and after heating were determined by SDS-PAGE gel. The results are as shown in FIG. 15.

### Conclusion:

The conjugate obtained without the addition of maleimide derivative small molecules has poor thermal stability, with a degradation rate reaching 50% after heating at 95°C for 10 minutes, while the protein-nucleic acid conjugates obtained by the method of the present invention with the addition of maleimide derivative small molecules have significantly improved thermal stability.

### Example 12. Test of protein substrate

In this example, the synthesis reaction of the present invention was tested for other proteins with a polyhistidine tag at the N-terminus. The proteins used in this example included a green fluorescent protein, a ubiquitin protein, and a small ubiquitin-like modifier protein.

The reaction steps of this example were the same as those of Example 6, in which the short peptide containing His tag was Met(His)₆, the azido-containing 2-picolinaldehyde derivative was 6-(azidomethyl)-4-methoxypicolinaldehyde, the maleimide derivative was 3-maleimidopropionic acid, and the modified nucleic acid was the dibenzocyclooctyne-modified nucleic acid prepared in Example 2.

### Experimental steps:

Step 1: 3 nmol protein was added to 30 µL of 50 mM phosphate buffer solution with pH 7.4, 30 nmol compound 2 (NLP, dissolved in 1 µL of DMSO, 60 nmol for small ubiquitin-like modifier protein) obtained in Example 1 and 3-maleimidopropionic acid (dissolved in 1 µL of DMSO, 120 nmol for green fluorescent protein, 15 nmol for ubiquitin protein, 30 nmol for small ubiquitin-like modifier protein) were added, and a reaction was performed at 37°C overnight in a PCR instrument.

Step 2: The reaction liquid was transferred to a 0.5 mL ultrafiltration tube and diluted to 500 µL by adding a phosphate buffer, the sample was concentrated to 30 µL by centrifugation at 12000 rpm for 10 minutes, and this process was repeated three times to remove excess small molecule compound to obtain a protein moiety-linker conjugate.

Step 3: 4.5 nmol nucleic acid with 3'-end DBCO modification and 5'-end biotin modification was added to the protein moiety-linker conjugate dissolved in 30 µL of 50 mM phosphate buffer solution with pH 7.4, and a reaction was performed at room temperature overnight to obtain a protein-nucleic acid conjugate. The proportion of the protein-nucleic acid conjugate was determined by SDS-PAGE gel. The results are as shown in FIG. 16.

### Conclusion:

Different proteins with polyhistidine tag at the N-terminus all can be conjugated to a nucleic acid according to the synthesis method of the present invention to obtain protein-nucleic acid conjugates, with high yields.

### Example 13. Test of conjugation compatibility with long-chain nucleic acid

In this example, the synthesis reaction of the present invention was tested for a long-chain nucleic acid of 65 bases in length with 3'-end DBCO modification and 5'-end biotin modification.

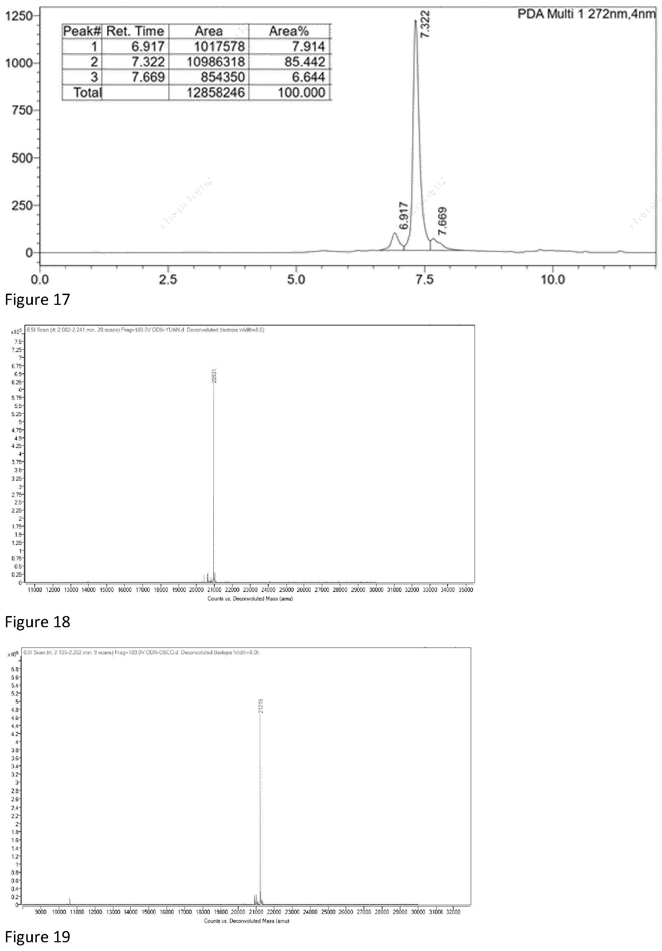

The method for synthesizing a long-chain oligonucleotide with 3'-end DBCO modification and 5'-end biotin modification was the same as that of Example 2. The reaction is as shown in FIG. 3. The HPLC analysis profile is as shown in FIG. 17. The mass spectrometry profiles are as shown in FIG. 18 and FIG. 19.

Sequence of long-chain nucleic acid with 5'-end biotin and 3'-end amino: 
<PCbt>AGGCGAACTGTTTTGGTCATAACCCGCTACTGATTGTTCGCACGGAGTGCTGTGCTTGTGTAAGG
<3am> (SEQ ID NO. 6)
 where <PCbt> is a 5'-end photocleavable biotin modification, and <3am> is a 3'-end amino modification (Genscript)

**Table 4. Molecular weights of test samples detected by mass spectrometry**

| Test sample | Detected molecular weight | Theoretical molecular weight |
|---|---|---|
| Long-chain oligonucleotide with 3'-end amino modification and 5'-end biotin modification | 20931 | 20932 |
| Long-chain oligonucleotide with 3'-end DBCO modification and 5'-end biotin modification | 21219 | 21220 |

### Experimental steps:

Step 1: 3 nmol protein was added to 30 µL of 50 mM phosphate buffer solution with pH 7.4, 30 nmol compound 2 (NLP, dissolved in 1 µL of DMSO, 60 nmol for small ubiquitin-like modifier protein) obtained in Example 1 and 3-maleimidopropionic acid (dissolved in 1 µL of DMSO, 120 nmol for green fluorescent protein, 15 nmol for ubiquitin protein, 30 nmol for small ubiquitin-like modifier protein) were added, and a reaction was performed at 37°C overnight in a PCR instrument.

Step 2: The reaction liquid was transferred to a 0.5 mL ultrafiltration tube and diluted to 500 µL by adding a phosphate buffer, the sample was concentrated to 30 µL by centrifugation at 12000 rpm for 10 minutes, and this process was repeated three times to remove excess small molecule compound to obtain a protein moiety-linker conjugate.

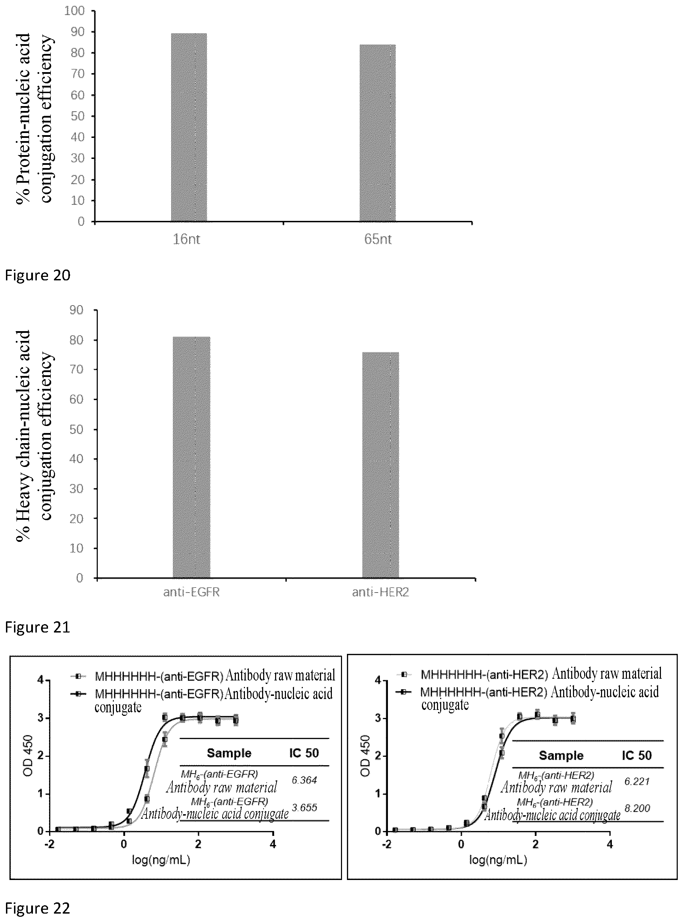

Step 3: 4.5 nmol long-chain nucleic acid with 3'-end DBCO modification and 5'-end biotin modification was added to the protein moiety-linker conjugate dissolved in 30 µL of 50 mM phosphate buffer solution with pH 7.4, and a reaction was performed at room temperature overnight to obtain a protein-nucleic acid conjugate. The proportion of the protein-nucleic acid conjugate product was determined by SDS-PAGE gel. The results are as shown in FIG. 20.

### Conclusion:

A longer nucleic acid molecule, e.g., a 65 nt nucleic acid molecule, can be conjugated to a protein moiety-linker conjugate according to the synthesis method of the present invention to obtain a protein-nucleic acid conjugate, with a high yield.

### Example 14. Test of antibody substrate

In this example, the synthesis reaction of the present invention was tested for antibodies with a polyhistidine tag at the N-terminus of the heavy chain. The antibodies used in this example included anti-EGFR and anti-HER2 antibodies.

The reaction steps of this example were the same as those of Example 6, in which the short peptide containing His tag was Met(His)₆, the azido-containing 2-picolinaldehyde derivative was 6-(azidomethyl)-4-methoxypicolinaldehyde, the maleimide derivative was 3-maleimidopropionic acid, and the modified nucleic acid was the dibenzocyclooctyne-modified nucleic acid prepared in Example 2.

### Experimental steps:

Step 1: 3 nmol antibody was added to 100 µL of 50 mM phosphate buffer solution with pH 7.4, 120 nmol compound 2 (NLP, dissolved in 4 µL of DMSO) obtained in Example 1 and 60 nmol 3-maleimidopropionic acid (dissolved in 1 µL of DMSO) were added, and a reaction was performed at 37°C overnight in a PCR instrument.

Step 2: The reaction liquid was transferred to a 0.5 mL ultrafiltration tube and diluted to 500 µL by adding a phosphate buffer, the sample was concentrated to 30 µL by centrifugation at 12000 rpm for 10 minutes, and this process was repeated three times to remove excess small molecule compound to obtain an antibody moiety-linker conjugate.

Step 3: 24 nmol nucleic acid with 3'-end DBCO modification and 5'-end biotin modification was added to the antibody moiety-linker conjugate dissolved in 30 µL of 50 mM phosphate buffer solution with pH 7.4, and a reaction was performed at room temperature overnight to obtain a protein-nucleic acid conjugate. The modification proportion of the heavy chain of the antibody-nucleic acid conjugate was determined by SDS-PAGE gel. The results are as shown in FIG. 21.

Step 4: The antibodies and the obtained antibody-nucleic acid conjugates were subjected to Elisa assay and the IC50 values were compared. The results are as shown in FIG. 22.

Antibody sequences:
Anti-EGFR antibody (Genscript)
   Heavy chain: (SEQ ID NO. 7) 
MHHHHHHQVQLKQSGPGLVQPSQSLSITCTVSGFSLTNYGVHWVRQSPGKGLEWLGVIWSGGNTDYNTPF
TSRLSINKDNSKSQVFFKMNSLQSNDTAIYYCARALTYYDYEFAYWGQGTLVTVSAASTKGPSVFPLAPSSKST
SGGTAALGCLVKDYFPEPVTVSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKSRWQQGNVFSCSV
MHEALHNHYTQKSLSLSPGK
   Light chain: (SEQ ID NO. 8) 
DILLTQSPVILSVSPGERVSFSCRASQSIGTNIHWYQQRTNGSPRLLIKYASESISGIPSRFSGSGSGTDFTLSINSV
ESEDIADYYCQQNNNWPTTFGAGTKLELKRTVAAPSVFIFPPSDEQLKSGTASVVCLLNNFYPREAKVQWKV
DNALQSGNSQESVTEQDSKDSTYSLSSTLTLSKADYEKHKVYACEVTHQGLSSPVTKSFNRGEC
Anti-HER2 antibody (Genscript)
   Heavy chain: (SEQ ID NO. 9) 
MHHHHHHEVQLVESGGGLVQPGGSLRLSCAASGFNIKDTYIHWVRQAPGKGLEWVARIYPTNGYTRYADSV
KGRFTISADTSKNTAYLQMNSLRAEDTAVYYCSRWGGDGFYAMDYWGQGTLVTVSSASTKGPSVFPLAPSS
KSTSGGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTISKAKGQPREPQVYTLP
PSREEMTKNQVSLTCLVKGFYPSDIAVEWESNGQPENNYKTTPPVLDSDGSFFLYSKLTVDKSRWQQGNVFS
CSVMHEALHNHYTQKSLSLSPGK
   Light chain: (SEQ ID NO. 10)

DIQMTQSPSSLSASVGDRVTITCRASQDVNTAVAWYQQKPGKAPKLLIYSASFLYSGVPSRFSGSRSGTDFTLT
ISSLQPEDFATYYCQQHYTTPPTFGQGTKVEIKRTVAAPSVFIFPPSDEQLKSGTASVVCLLNNFYPREAKVQW
KVDNALQSGNSQESVTEQDSKDSTYSLSSTLTLSKADYEKHKVYACEVTHQGLSSPVTKSFNRGEC

### Conclusion:

Antibodies having polyhistidine tag at the N-terminus of the heavy chain can be conjugated to a nucleic acid according to the synthesis method of the present invention to obtain antibody-nucleic acid conjugates, with high yields and without affecting the antigen-binding ability of the antibodies themselves.

The embodiments of the present invention are not limited to those described in the above examples, and various modifications and improvements in forms and details may be made to the present invention by those of ordinary skill in the art without departing from the spirit and scope of the present invention, and these modifications and improvements are considered to fall within the scope of protection of the present invention.

## Claims

1. A protein moiety-linker conjugate having a structure as shown in formula (I): 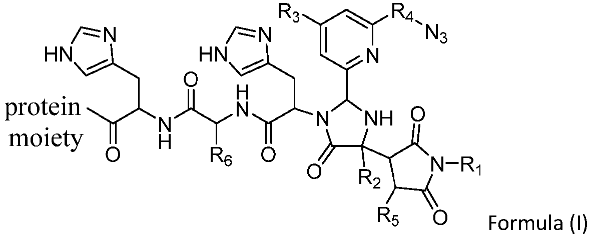
wherein R₁ is selected from hydrogen, hydrocarbyl, amine group, amino, hydroxyl, carboxyl, the hydrocarbyl optionally has one or more substituents selected from hydrocarbyl, amine group, amino, hydroxyl, carboxyl, and the hydrocarbyl is preferably alkyl, aryl, or a combination thereof, wherein the alkyl optionally has one or more identical or different substituents selected from aryl, amine group, amino, hydroxyl, carboxyl, and the aryl optionally has one or more identical or different substituents selected from alkyl, amine group, amino, hydroxyl, carboxyl, or R₁ is selected from a fluorophore, a PEG modification group, a biotin modification group, a polypeptide modification group, a nucleic acid modification group;
R₂ is selected from a side chain moiety of any one of natural amino acids;
R₃ is selected from hydrogen, alkyl, alkoxy, hydroxyl, amino, amine group;
R₄ is selected from alkylene, arylene, PEG;
R₅ is selected from hydrogen and alkyl;
R₆ is selected from a side chain moiety of any one of natural amino acids.

2. The protein moiety-linker conjugate according to claim 1, having the structure of:

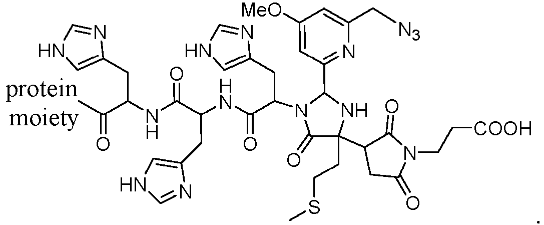

3. The protein moiety-linker conjugate according to claim 1 or 2, wherein the protein moiety is an antibody or a fragment thereof.

4. The protein moiety-linker conjugate according to any one of claims 1-3, wherein the protein moiety has a molecular weight ranging from 8 kDa to 160 kDa.

5. A method for preparing the protein moiety-linker conjugate according to any one of claims 1-4, comprising the steps of:
(1) modifying the N-terminus of a protein using a short peptide containing His tag to obtain a modified protein;
(2) subjecting the modified protein to a reaction with an azido-containing 2-picolinaldehyde derivative and a maleimide derivative to obtain the protein moiety-linker conjugate.

6. The preparation method according to claim 5, wherein the short peptide containing His tag has a sequence of Xaa-His-Xaa-His, and Xaa is any one of natural amino acid residues.

7. The preparation method according to claim 5 or 6, wherein the azido-containing 2-picolinaldehyde derivative is a 6-(azidomethyl)-2-picolinaldehyde derivative, preferably 6-(azidomethyl)-4-methoxypicolinaldehyde.

8. The method according to any one of claims 5-7, wherein the maleimide derivative has the structure of: 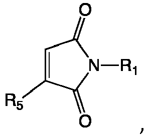
wherein R₁ is selected from hydrogen, hydrocarbyl, amine group, amino, hydroxyl, carboxyl, the hydrocarbyl optionally has one or more substituents selected from hydrocarbyl, amine group, amino, hydroxyl, carboxyl, and the hydrocarbyl is preferably alkyl, aryl, or a combination thereof, wherein the alkyl optionally has one or more identical or different substituents selected from aryl, amine group, amino, hydroxyl, carboxyl, and the aryl optionally has one or more identical or different substituents selected from alkyl, amine group, amino, hydroxyl, carboxyl, or R₁ is a fluorophore, a PEG modification group, a biotin modification group, a polypeptide modification group, a nucleic acid modification group;
R₅ is selected from hydrogen and alkyl.

9. The method according to any one of claims 5-8, wherein the maleimide derivative is selected from

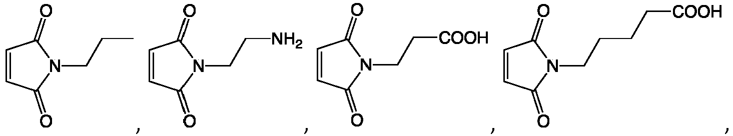

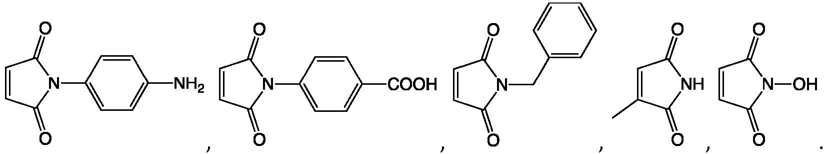

10. The method according to any one of claims 5-9, wherein the modified protein, the azido-containing 2-picolinaldehyde derivative and the maleimide derivative react in a molar ratio of 1 : 5-60 : 5-40.

11. The method according to any one of claims 5-10, wherein the molar ratio of the azido-containing 2-picolinaldehyde derivative to the maleimide derivative is in the range of 30 : 1 to 1 : 1.

12. The method according to any one of claims 5-11, wherein the reaction of the modified protein with the azido-containing 2-picolinaldehyde derivative and the maleimide derivative is performed at pH 6.0-9.0.

13. Use of the protein moiety-linker conjugate according to any one of claims 1-4 in the preparation of a protein-active molecule conjugate.

14. The use according to claim 13, wherein the active molecule is selected from a nucleic acid molecule, a fluorescent molecule, a PEG molecule, a biotin molecule, a polypeptide molecule, a small drug molecule.

15. A method for synthesizing a protein-active molecule conjugate, comprising the steps of:
subjecting the protein moiety-linker conjugate according to any one of claims 1-4 to a reaction with an alkynyl-modified active molecule to obtain the protein-active molecule conjugate.

16. The method for synthesizing a protein-active molecule conjugate according to claim 15, wherein the active molecule is selected from a nucleic acid molecule, a fluorescent molecule, a PEG molecule, a biotin molecule, a polypeptide molecule, a small drug molecule.

17. The method for synthesizing a protein-active molecule conjugate according to claim 15 or 16, wherein the active molecule is a nucleic acid molecule.

18. The method for synthesizing a protein-active molecule conjugate according to claim 15 or 16, wherein the alkynyl-modified active molecule is a nucleic acid molecule with an alkynyl-containing group modification at the 3' end, the 5' end, or any position between the two ends.

19. The method for synthesizing a protein-active molecule conjugate according to claim 15 or 16, wherein the alkynyl-modified active molecule is a nucleic acid molecule with a dibenzocyclooctyne modification at the 3' end or the 5' end or any position between the two ends.

20. The method for synthesizing a protein-active molecule conjugate according to any one of claims 17-19, wherein the nucleic acid molecule is selected from natural DNA; natural RNA; a nucleic acid having a base modification such as 2'-F modification, 2'-OMe modification, and 2'-MOE modification; a nucleic acid having a biotin modification, a fluorophore modification, a polypeptide modification, a PEG modification, a phosphorylation modification; the nucleic acid molecule includes a single-stranded nucleic acid or a double-stranded nucleic acid.

21. The method for synthesizing a protein-active molecule conjugate according to any one of claims 17-20, wherein the nucleic acid molecule has a length of 10 to 100 nt.

22. A protein-active molecule conjugate obtained by the method for synthesizing a protein-active molecule conjugate according to any one of claims 15-20.

23. The protein-active molecule conjugate according to claim 22, wherein the active molecule is selected from a nucleic acid molecule, a fluorescent molecule, a PEG molecule, a biotin molecule, a polypeptide molecule, a small drug molecule.

24. The protein-active molecule conjugate according to claim 23, wherein the active molecule is a nucleic acid molecule.

25. A protein-nucleic acid conjugate, having the structure of: 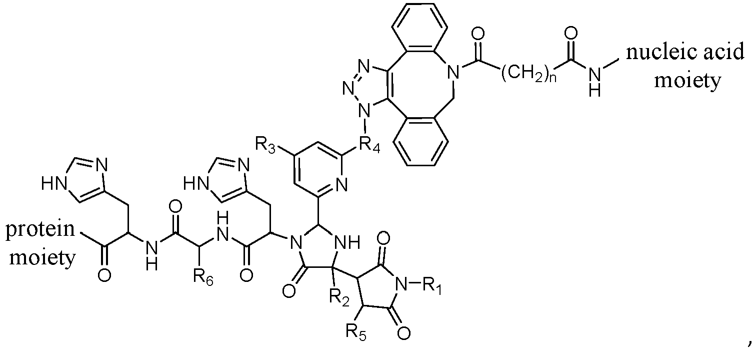
wherein R₁ is selected from hydrogen, hydrocarbyl, amine group, amino, hydroxyl, carboxyl, the hydrocarbyl optionally has one or more substituents selected from hydrocarbyl, amine group, amino, hydroxyl, carboxyl, and the hydrocarbyl is preferably alkyl, aryl, or a combination thereof, wherein the alkyl optionally has one or more identical or different substituents selected from aryl, amine group, amino, hydroxyl, carboxyl, and the aryl optionally has one or more identical or different substituents selected from alkyl, amine group, amino, hydroxyl, carboxyl, or R₁ is selected from a fluorophore, a PEG modification group, a biotin modification group, a polypeptide modification group, a nucleic acid modification group;
R₂ is selected from a side chain moiety of any one of natural amino acids;
R₃ is selected from hydrogen, alkyl, alkoxy, hydroxyl, amino, amine group;
R₄ is selected from alkylene, arylene, PEG;
R₅ is selected from hydrogen and alkyl;
R₆ is selected from a side chain moiety of any one of natural amino acids;
n is an integer selected from 1-8.

26. The protein-nucleic acid conjugate according to claim 25, having the structure of:

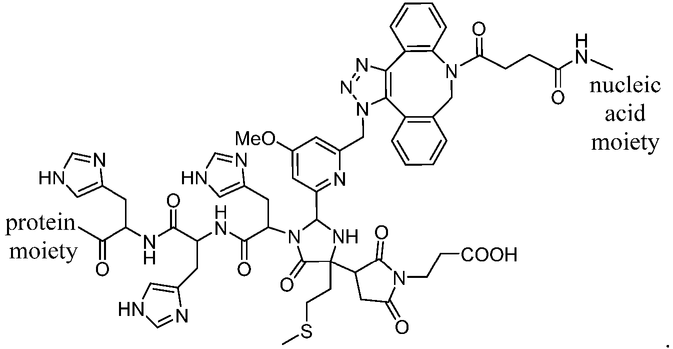

27. The protein-nucleic acid conjugate according to claim 25 or 26, wherein the protein moiety is an antibody or a fragment thereof.

28. The protein-nucleic acid conjugate according to any one of claims 25-27, wherein the protein moiety has a molecular weight ranging from 8 kDa to 160 kDa.

29. The protein-nucleic acid conjugate according to any one of claims 25-28, wherein the nucleic acid molecule is selected from natural DNA; natural RNA; a nucleic acid having a base modification such as 2'-F modification, 2'-OMe modification, and 2'-MOE modification; a nucleic acid having a biotin modification, a fluorophore modification, a polypeptide modification, a PEG modification, a phosphorylation modification; the nucleic acid molecule includes a single-stranded nucleic acid or a double-stranded nucleic acid.

30. The protein-nucleic acid conjugate according to any one of claims 25-29, wherein the nucleic acid molecule has a length of 10 to 100 nt.
